# EUROPÄISCHE PATENTSCHRIFT

(11) **EP 2 252 393 B1**
(45) Veröffentlichungstag und Bekanntmachung des Hinweises auf die Patenterteilung: **30.04.2014**
(21) Anmeldenummer: 09720980.3
(22) Anmeldetag: 16.02.2009
(51) Int. Cl.: B01J 20/29

(54) **NEUE CHIRALE SELEKTOREN UND STATIONÄRE PHASEN ZUR TRENNUNG VON ENANTIOMERENGEMISCHEN**
NOVEL CHIRAL SELECTORS AND STATIONARY PHASES FOR SEPARATING ENANTIOMER MIXTURES
NOUVEAUX SÉLECTEURS CHIRAUX ET PHASES STATIONNAIRES POUR SÉPARATION DE MÉLANGES D'ÉNANTIOMÈRES

(30) Priorität: 10.03.2008 DE 102008013500
(43) Veröffentlichungstag der Anmeldung: 24.11.2010
(73) Patentinhaber: Evonik Degussa GmbH, 45128 Essen (DE)
(72) Erfinder: KUNZ, Franz-Rudolf, 63571 Gelnhausen (DE); RICHTER, Peter, 63791 Karlstein (DE); MERGET, Stefan, 63110 Rodgau (DE); SINGER, Roland, 63755 Alzenau (DE); MÜLLER, Thomas, 63486 Bruchköbel (DE)
(86) Internationale Anmeldenummer: PCT/EP2009/051745
(87) Internationale Veröffentlichungsnummer: WO 2009/112325

(56) Entgegenhaltungen:
- SCHLEIMER, MICHAEL ET AL: "Enantiomer separation by high-performance liquid chromatography on polysiloxane-based chiral stationary phases" JOURNAL OF CHROMATOGRAPHY, A , 679(1), 23-34 CODEN: JCRAEY; ISSN: 0021-9673, 1994, XP009115160
- WELCH C J: "EVOLUTION OF CHIRAL STATIONARY PHASE DESIGN IN THE PIRKLE LABORATORIES" JOURNAL OF CHROMATOGRAPHY, ELSEVIER SCIENCE PUBLISHERS B.V. AMSTERDAM, NL, Bd. 666, Nr. 1/02, 22. April 1994 (1994-04-22), Seiten 3-26, XP000482399 ISSN: 0021-9673
- PIERKLE ET AL: "An Improved Chiral Stationary Phase For The Facile Separation Of Enantiomers" JOURNAL OF CHROMATOGRAPHY, Bd. 441, 1988, Seiten 311-322, XP009115155 in der Anmeldung erwähnt
- PIRKLE W H ET AL: "IMPROVED CHIRAL STATIONARY PHASE FOR THE SEPARATION OF THE ENANTIOMERS OF CHIRAL ACIDS AS THEIR ANILIDE DERIVATIVES" JOURNAL OF CHROMATOGRAPHY, ELSEVIER SCIENCE PUBLISHERS B.V. AMSTERDAM, NL, Bd. 471, Nr. 1, 2. Juni 1989 (1989-06-02), Seiten 271-281, XP000026456 ISSN: 0021-9673

## Beschreibung

Die vorliegende Erfindung betrifft die Verwendung von in α-Position unsubstituierten β-Aminosäuren und insbesondere ihren Derivaten als chirale Selektoren zur Trennung von Substanzgemischen, bevorzugt von Gemischen chiraler Substanzen, besonders bevorzugt von Enantiomerengemischen, insbesondere von Enantiomeren von Substanzen ausgewählt aus der Gruppe enthaltend β-Aminosäuren und ihre Derivate, α-Aminosäuren und α-Hydroxysäuren, sowie chirale stationäre Phasen (CSP), die in α-Position unsubstituierte β-Aminosäuren und insbesondere ihre Derivate als zentrale chirale Selektoren enthalten, ihre Synthese und Verfahren zur Trennung von Substanzgemischen, bevorzugt von Gemischen chiraler Substanzen, besonders bevorzugt von Enantiomerengemischen, insbesondere von Enantiomeren von Substanzen ausgewählt aus der Gruppe enthaltend β-Aminosäuren und ihre Derivate, α-Aminosäuren und α-Hydroxysäuren mit Hilfe dieser stationärer Phasen.

Der steigende Bedarf an enantiomerenreinen Substanzen und Wirkstoffen für chemische und pharmazeutische Anwendungen hat zur Entwicklung einer Vielzahl stereoselektiver Trenntechnologien geführt, vor allem im Bereich der Chromatographie, die sowohl im analytischen Maßstab zur Kontrolle der Enantiomerenreinheit und Überprüfung von Racemisierungsprozessen, zur pharmazeutischen Qualitätskontrolle und für pharmakokinetische Untersuchungen wie auch im präparativen Maßstab zur Bereitstellung enantiomerenreiner Verbindungen eingesetzt werden können.

Enantiomere weisen in einer achiralen Umgebung im Gegensatz zu Diastereomeren identische chemische und physikalische Eigenschaften auf. Chromatographische Enantiomerentrennungen können daher entweder unter Verwendung indirekter Methoden durchgeführt werden, d. h. nach Umsetzung des Analyten mit einem chiralen Derivatisierungsreagenz zu einem Diastereomerengemisch, welches sich im Gegensatz zu einem Enantiomerengemisch an achiralem Phasenmaterial trennen läßt oder mit Hilfe sogenannter direkter Methoden unter Verwendung eines chiralen Selektors, der in die mobile oder stationäre Phase inkorporiert wird. Hier beruht die Trennleistung auf der unterschiedlichen Stabilität der diastereomeren Komplexe, die durch nicht-kovalente Wechselwirkungen zwischen dem Analyten und dem Selektor gebildet werden.

Direkte wie indirekte Verfahren fanden bereits in diversen chromatographischen und elektrophoretischen Methoden wie der Gaschromatographie (GC), der Hochleistungsflüssigkeitschromatographie (auch Hochdruckflüssigkeitschromatographie, HPLC), der Dünnschichtchromatographie (DC), der Super- und Subkritischen Flüssigchromatographie (SFC), der Kapillarelektrochromatographie (CEC) und der Kapillarelektrophorese (CE) Anwendung im Bereich der Enantiomerentrennung (Gübitz und Schmid (Eds.), Methods in Molecular Biology, Vol 243: Chiral Separations: Methods and Protocols, Humana Press Inc: Totowa, NJ: 2004; Gübitz und Schmid, Biopharm. Drug Dispos. 2001, 22, 291-336).

Eine Derivatisierung des Analyten bedeutet mindestens einen zusätzlichen Reaktionsschritt, der zur Bildung unerwünschter Neben- und Zersetzungsprodukte sowie zur (partiellen) Racemisierung führen kann. Darüber hinaus müssen im Analyten zur Derivatisierung geeignete funktionelle Gruppen vorhanden sein, und das chirale Derivatisierungsreagenz muss in hoher Enantiomerenreinheit verfügbar sein (Gübitz und Schmid, Biopharm. Drug Dispos. 2001, 22, 291-336), weshalb heutzutage nicht-derivative direkte chromatographische oder elektrophoretische Methoden bevorzugt werden.

Der Zusatz eines chiralen Selektors zur mobilen Phase eines chromatographischen oder elektrophoretischen Systems stellt zwar eine in der Handhabung simple Methode zur Enantiomerentrennung dar, ist jedoch sehr kostenintensiv und nicht in allen Fällen praktikabel.

Direkte chromatographische Verfahren unter Verwendung chiraler stationärer Phasen, in denen ein chiraler Selektor kovalent oder adsorptiv an ein Trägermaterial gebunden ist, sind in der Handhabung komfortabel, und - eine hinreichende Trennleistung des chiralen Phasenmaterials vorausgesetzt - auch im präparativen Maßstab anwendbar. Gerade im Bereich der chiralen stationären Phasen ist ein chiraler Selektor wünschenswert, der zwar einerseits eine effiziente Trennung der beiden Enantiomere einer chiralen Verbindung erlaubt, andererseits jedoch flexibel genug ist, um die Anwendung für eine breite Klasse von Verbindungen zu erlauben.

Eine Variante der direkten Methoden sind Ligandenaustauschverfahren (LE), denen die Bildung ternärer gemischter Komplexe zwischen einem Metallion, einem chiralen Selektor und dem Analyten, die beide als Liganden am Metallion fungieren, zugrunde liegt. Verantwortlich für eine erfolgreiche Trennung sind hier die unterschiedlichen Stabilitätskonstanten der gemischten Komplexe mit dem (R)- bzw. (S)-Enantiomer des Analyten. Das Ligandenaustauschprinzip konnte in einer Reihe der oben genannten Verfahren erfolgreich zur Enantiomerentrennung eingesetzt werden: zum einen unter Zusatz eines chiralen Selektors zum Elektrolyten in der Kapillarelektrophorese und darüber hinaus unter Verwendung chiraler stationärer Phasen in der klassischen Säulenchromatographie, der Hochleistungsflüssigchromatographie, der Dünnschichtchromatographie und der Kapillarelektrochromatographie, wobei der chirale Selektor in diesen Fällen kovalent oder adsorptiv an das Trägermaterial gebunden sein kann.

Da sich allerdings chirale Trennungen bis heute nicht vorhersagen lassen, ist es nach wie vor eine große chromatographische Herausforderung, die geeignete Kombination von chiraler stationärer und mobiler Phase effizient und schnell zu finden (Subramanian, Practical Approach to Chiral Separations by Liquid Chromatography, Wiley-VCH: Weinheim 1994; Gübitz und Schmid (Eds.), Methods in Molecular Biology, Vol 243: Chiral Separations: Methods and Protocols, Humana Press Inc: Totowa, NJ: 2004; Francotte, GIT Labor-Fachzeitschrift 5/2006, 452-455), und intensive Forschungen haben zu stets neuen und verbesserten Phasenmaterialien geführt (Lämmerhofer und Lindner, In: Separation Methods in Drug Synthesis and Purification, Valkó (Ed.), Elsevier: Amsterdam 2000, 337-437). Gemäß Armstrong et al. (Anal. Chem. 2001, 73, 557A-561 A) waren bereits 2001 mehr als 100 chirale stationäre Phasen auf der Basis verschiedener Selektoren allein für die Hochleistungsflüssigkeitschromatographie kommerziell erhältlich. Jeder Hersteller bietet umfangreiche Applikations-Handbücher an, in denen fast ausschließlich produktspezifisch die vielfältigsten Trennbedingungen z.B. für aliphatische, aromatische, alicyclische oder heterocyclische chirale Amine, Alkohole, Aminoalkohole oder α-Aminosäuren und deren Derivate aufgelistet sind. Diese Vielzahl an kommerziell erhältlichen chiralen stationären Phasen belegt zum einen die immense Bedeutung und das große Interesse an diesen Trenntechniken, verdeutlicht jedoch auch einen großen Nachteil, der den direkten chromatographischen und elektrophoretischen Methoden mit Hilfe chiraler stationärer Phasen bis heute anhaftet: Oftmals ist eine Reihe (teurer) chiraler stationärer Phasen notwendig, um eine effiziente Trennung selbst bei strukturell eng verwandten Bausteinen zu erzielen.

So belegen eigene Untersuchungen wie auch Literaturangaben, dass bislang zur chiralen Chromatographie beispielsweise von β-Aminosäuren und ihren Derivaten eine Vielzahl stationärer Phasen notwendig ist, die dann gaschromatographisch (GC) oder flüssigchromatographisch (bspw. HPLC) eingesetzt werden. Aus dem Stand der Technik sind z.B. Cellulosecarbamat-Phasen, Amylose-Derivate, Kronenether (Berkecz et al., J. Chromatogr. A, 2006, 1125, 138-143 ; Hyun et al., J. Sep. Sci. 2005, 28, 421-427), Ligandenaustausch-Phasen (Hyun et al., J. Sep. Sci. 2003, 26, 1615-1622; Hyun et al., Biomed. Chromatogr. 2003, 17, 292-296) oder makrozyklische Glycopeptid-Phasen (Sztojkov-Ivanov et al., Chromatographia 2006, 64, 89-94; Illisz et al., J. Sep. Sci. 2006, 29, 1305-1321 und dort zitierte Literatur; D' Acquarica et al., Tetrahedron: Asymmetry 2000, 11, 2375-2385) bekannt.

β-Aminosäuren sind in den letzten Jahren aufgrund ihrer besonderen pharmakologischen Eigenschaften als Schlüsselkomponenten in eine Vielzahl von Peptidomimetika und weiteren biologisch aktiven Substanzen eingebaut worden (Kuhl et al., Amino Acids 2005, 29, 89-100). Damit verbunden entwickelte sich auch ein steigender Bedarf an analytischen Methoden zur Prüfung der Enantiomerenreinheit der Synthesebausteine und Endprodukte, gerade auch im Bereich der Minorspurenbestimmung zum Nachweis von Spuren eines Enantiomers in Gegenwart eines deutlichen Überschusses der optischen Antipode (Juaristi und Soloshonok, Enantioselective Synthesis of β-Amino Acids, Wiley-VCH: New York 2005).

Die Aufgabe der vorliegenden Erfindung besteht darin, neue chirale Selektoren bereitzustellen, auf Grundlage derer neue chirale Phasen zur Trennung von Substanzgemischen, bevorzugt von Gemischen chiraler Substanzen, besonders bevorzugt von Enantiomerengemischen, insbesondere von Enantiomeren von Substanzen ausgewählt aus der Gruppe enthaltend β-Aminosäuren und ihre Derivate, α-Aminosäuren und α-Hydroxysäuren, bereitgestellt werden können, die eine effiziente und möglichst universelle Auftrennung von Enantiomerenpaaren chiraler Verbindungen, insbesondere von β-Aminosäuren und ihren Derivaten mit chromatographischen Methoden, insbesondere mit Hilfe der Hochleistungsflüssigkeitschromatographie im analytischen und präparativen Maßstab erlauben.

Völlig überraschender Weise ist nun gefunden worden, dass in α-Position unsubstituierte β-Aminosäurederivate flexible und dennoch hoch selektive chirale Selektoren in Verfahren zur Trennung von Substanzgemischen, bevorzugt von Gemischen chiraler Substanzen, besonders bevorzugt von Enantiomerengemischen, insbesondere von β-Aminosäuren und ihren Derivaten darstellen. Weitere Substanzklassen, bei denen die erfindungsgemäßen Selektoren zur Enantiomerentrennung verwendet werden können, sind beispielsweise α-Amino- und α-Hydroxysäuren. Ein besonderer Vorteil der vorliegenden Erfindung ist somit, dass die chiralen Phasen, die chirale Selektoren auf der Basis von α-Position unsubstituierten β-Aminosäurederivaten enthalten, überraschenderweise universell zur chromatographischen Trennung einer großen Anzahl von Substanzklassen einsetzbar sind.

Ein Gegenstand der vorliegenden Erfindung ist daher die Verwendung chiraler Selektoren der Struktur (I) 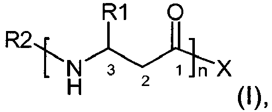 worin n = 1-5, bevorzugt n = 1 oder n = 2, R¹ = (C₁-C₄)-Alkyl, (C₆-C₁₀)-Aryl, (C₇-C₁₃)-Aralkyl, (C₇-C₁₀)-Heteroaralkyl, Pyridyl, Hydroxymethyl, CH(OH)CH₃, CH₂CONH₂, CH₂COOH, (CH₂)₂CONH₂, (CH₂)₂COOH, (CH₂)₄NH₂, (CH₂)₂SCH₃ oder (CH₂)₃NHC(NH)NH₂;
wobei entweder X = OH ist oder einen Aminopropyllinker darstellt, über den der Selektor als Carbonsäureamid an das Trägermaterial gebunden ist,
und R² = 3,5-Dinitrobenzoyl oder Naphthyl bedeutet, wenn der chirale Selektor über einen Linker X kovalent an ein Trägermaterial gebunden wird,
oder R² = CH₂CHR³R⁴ bedeutet, worin R³ = H oder OH und R⁴ = (C₁-C₂₀)-Alkyl, (C₆-C₁₀)-Aryl oder (C₇-C₁₃)-Aralkyl bedeutet, wenn X = OH darstellt.

Erfindungsgemäß liegt der chirale Selektor überwiegend in einer absoluten Konfiguration bezüglich des Kohlenstoffatomes C3 (β-Kohlenstoffatom) vor, während ein in den Seitenketten potentiell vorhandenes Stereozentrum nicht in überwiegend einer absoluten Konfiguration vorliegen muss.

Ein (C₁-C₄)-Alkylrest bezeichnet im Sinne der Erfindung einen Rest mit 1 bis 4 gesättigten Kohlenstoffatomen, der beliebige Verzweigungen aufweisen kann, bevorzugt die Reste Methyl, Isopropyl, Isobutyl und sec-Butyl.

Ein (C₆-C₁₀)-Arylrest bezeichnet im Rahmen der Erfindung einen aromatischen Rest mit 6 bis 10 Kohlenstoffatomen, bevorzugt die Reste Phenyl sowie 1- und 2-Naphthyl, die mit weiteren Resten oder funktionellen Gruppen mono-, oligo-, poly-oder persubstituiert sein können, insbesondere mit Fluor-, Chlor-, Brom-, Methyl-und/oder Trifluormethylresten sowie Hydroxy- und/oder Cyanogruppen.

Ein (C₇-C₁₃)-Aralkylrest bezeichnet im Sinne der Erfindung bevorzugt einen über eine Methylengruppe an das Molekül gebundenen (C₆-C₁₀)-Arylrest, sowie des weiteren bevorzugt Phenylethyl- und Diphenylmethylreste.

Ein (C₇-C₁₀)-Heteroaralkylrest bezeichnet im Sinne der Erfindung bevorzugt einen über eine Methylengruppe an das Molekül gebundenen Pyridyl- oder Indolylrest.

Ein Napthylrest bezeichnet im Sinne der Erfindung bevorzugt einen 1-Naphthyl oder 2-Naphthylrest, an den weitere Substituenten gebunden sein können.

Bevorzugt bedeutet R¹ = Phenyl, besonders bevorzugt ist die Ausführungsform, in der R¹ = Phenyl und n = 1 oder n = 2 bedeutet. Bevorzugt bedeutet des weiteren R⁴ = Phenyl.

Gegenstand der Erfindung ist weiterhin die Verwendung der durch Struktur (I) beschriebenen chiralen Selektoren zur Herstellung stationärer Phasen für chromatographische Trennverfahren von Substanzgemischen, bevorzugt von Gemischen chiraler Substanzen, besonders bevorzugt von Enantiomerengemischen, insbesondere von Enantiomeren von Substanzen ausgewählt aus der Gruppe enthaltend β-Aminosäuren und ihre Derivate, α-Aminosäuren und α-Hydroxysäuren, wobei der chirale Selektor kovalent oder adsorptiv an ein Trägermaterial auf Kieselgel- oder Monolithbasis gebunden wird.

Die durch Struktur (I) beschriebenen chiralen Selektoren bzw. die auf ihnen basierenden chiralen stationären Phasen eignen sich als Selektoren in Verfahren zur chromatographischen Trennung von Substanzgemischen, bevorzugt von Gemischen chiraler Substanzen, besonders bevorzugt von Enantiomerengemischen, insbesondere von Enantiomeren von Substanzen ausgewählt aus der Gruppe enthaltend β-Aminosäuren und ihre Derivate, α-Aminosäuren und α-Hydroxysäuren. Die Probe kann hierbei neben dem Enantiomerengemisch auch weitere Verbindungen enthalten wie beispielsweise Nebenprodukte und/oder Verunreinigungen, die abgetrennt werden sollen. Chromatographische Verfahren im Sinne der Erfindung sind unter anderen die Dünnschichtchromatographie (DC), die Kapillarelektrochromatograhie (CEC), und besonders bevorzugt die Hochleistungsflüssigchromatographie (HPLC). Weitere chromatographische Verfahren im Sinne der Erfindung sind die Mikro-Hochleistungsflüssigchromatographie (µ-HPLC) und die Kapillarelektrochromatograhie (CEC) an Mikrochips (sogenannte lab-on-chip-Technologie). Ein besonderer Vorteil der vorliegenden Erfindung liegt darin, dass die chiralen Selektoren die Herstellung stationärer Phasen erlauben, die unter diversen Bedingungen stabil sind und sowohl im Normalphasenmodus (NP), wie auch im Polar-Organischen (PO) und im Umkehrphasenmodus (RP) betrieben werden können. Ein weiterer Vorteil der vorliegenden Erfindung besteht darin, dass die chiralen Selektoren aufgrund ihrer hohen Selektivität die Minorspurenbestimmung von Enantiomeren erlauben. Bevorzugt sind weiterhin die genannten chromatographischen Verfahren im Ligandenaustausch-Modus, bei denen die mobile Phase positiv geladene Metallionen, bevorzugt zweiwertige Metallionen, insbesondere Kupfer(II)ionen enthält (Davankov, J. Chromatogr. 1971, 60, 280-283). Im Ligandenaustausch-Modus ist die adsorptive Anbindung des chiralen Selektors gemäß Struktur (I) bevorzugt, wobei X = OH und R² = CH₂CHR³R⁴ bedeutet, worin R³ = H oder OH und R⁴ = (C₁-C₂₀)-Alkyl, (C₆-C₁₀)-Aryl oder (C₇-C₁₃)-Aralkyl darstellt.

Die Erfindung stellt des weiteren eine chirale stationäre Phase zur chromatographischen Trennung von Substanzgemischen, bevorzugt von Gemischen chiraler Substanzen, besonders bevorzugt von Enantiomerengemischen, insbesondere von Enantiomeren von Substanzen ausgewählt aus der Gruppe enthaltend β-Aminosäuren und ihre Derivate, α-Aminosäuren und α-Hydroxysäuren, beinhaltend ein Trägermaterial und einen chiralen Selektor zur Verfügung, wobei der chirale Selektor ein in α-Position unsubstituiertes β-Aminosäurederivat der Struktur 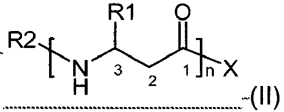 beinhaltet, worin n = 1-5, bevorzugt n = 1 oder n = 2, R¹ = (C₁-C₄)-Alkyl, (C₆-C₁₀)-Aryl, (C₇-C₁₃)-Aralkyl, (C₇-C₁₀)-Heteroaralkyl, Pyridyl, Hydroxymethyl, CH(OH)CH₃, CH₂CONH₂, CH₂COOH, (CH₂)₂CONH₂, (CH₂)₂COOH, (CH₂)₄NH₂, (CH₂)₂SCH₃ oder (CH₂)₃NHC(NH)NH₂, R² = 3,5-Dinitrobenzoyl oder Naphthylbedeutet und X einen Amino-propyllinker darstellt, über den der Selektor als Carbonsäureamid an das Trägermaterial gebunden ist.

Erfindungsgemäß liegt der chirale Selektor überwiegend in einer absoluten Konfiguration bezüglich des Kohlenstoffatomes C3 (β-Kohlenstoffatom) vor.

Bevorzugt bedeutet R¹ = Phenyl, besonders bevorzugt ist die Ausführungsform, in der R¹ = Phenyl und n = 1 oder n = 2 bedeutet.

Der Linker X stellt einen Aminopropyllinker dar, über den der Selektor als Carbonsäureamid an das Trägermaterial gebunden wird. Diese Ausführungsform ist in Struktur (IIa) dargestellt.

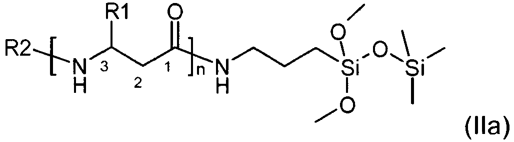

Bevorzugte Trägermaterialien im Sinne der Erfindung sind Materialien auf Kieselgel- oder Monolithbasis.

Die Erfindung stellt weiterhin ein Verfahren zur Herstellung einer chiralen stationären Phase enthaltend einen chiralen Selektor der Struktur (II) oder (IIa) bereit, umfassend die folgenden Schritte: (ia)Acylierung (R² = 3,5-Dinitrobenzoyl) einer in α-Position unsubstituierten β-Aminosäure oder des entsprechenden β-Aminosäureesters mit einem zur Acylierung geeigneten 3,5-Dinitrobenzoylderivat, bevorzugt 3,5-Dinitrobenzoylchlorid *oder (ib)* Synthese einer in α-Position unsubstituierten *N-*naphthylierten β-Aminosäure oder des entsprechenden β-Aminosäureesters (R² = Naphthyl) (ii) gegebenenfalls Verseifung der Esterfunktion, (iii) gegebenenfalls Kupplung weiterer in α-Position unsubstituierter β-Aminosäureeinheiten, (iv) kovalente Anbindung des chiralen Selektors an ein Trägermaterial, gegebenenfalls über einen Linker. Die Herstellung von Verbindungen der allgemeinen Struktur (II) und (IIa) kann beispielsweise durch Acylierung (R² = 3,5-Dinitrobenzoyl) der entsprechenden β-Amino-säure bzw. des entsprechenden β-Aminosäureesters mit einem zur Acylierung geeigneten 3,5-Dinitrobenzoylderivat, bevorzugt 3,5-Dinitrobenzoylchlorid (analog dem von Pirkle *et al.* publizierten Verfahren zur Darstellung chiraler Phasen u. a. auf der Basis von α-Aminosäuren (J. Chromatogr. 1980, 192, 143-158)) erfolgen. Die Synthese einer in α-Position unsubstituierten N-naphthylierten β-Aminosäure oder des entsprechenden β-Aminosäureesters in überwiegend einer enantiomeren Form (R² = Naphthyl) ist beispielsweise durch eine Variation der klassischen Bucherer Reaktion ausgehend von der entsprechenden β-Aminosäure und Naphthol (analog dem von Pirkle und Pochapsky publizierten Verfahren zur Herstellung von N-(2-Naphthyl)-2-aminosäuren und -estern mit hohen Enantiomerenreinheiten (J. Org. Chem. 1986, 51, 102-105)) möglich. Die β-Aminosäureester werden zum Aufbau der Selektoren mit repetitiver β-Aminosäureeinheit (n > 1) benötigt, der beispielsweise auf dem Wege der klassischen Peptidsynthese in Lösung erfolgen kann. Für Details und alternative Synthesewege sei auf die einschlägig bekannte Literatur zur Peptidsynthese verwiesen (z. B. Houben-Weyl, Methoden der organischen Chemie, Band 15/1 und 15/2; M. Bodanszky, Principles of Peptide Synthesis, Springer Verlag 1984).

Des weiteren stellt die Erfindung ein Verfahren zur chromatographischen Trennung von Substanzgemischen, bevorzugt von Gemischen chiraler Substanzen, besonders bevorzugt von Enantiomerengemischen, insbesondere von Enantiomeren von Substanzen ausgewählt aus der Gruppe enthaltend β-Aminosäuren und ihre Derivate, α-Aminosäuren und α-Hydroxysäuren, unter Verwendung der durch die Strukturen (II) oder (IIa) beschriebenen chiralen Selektoren oder der auf ihnen basierenden chiralen stationären Phasen bereit, beinhaltend (i) das In-Kontakt-Bringen einer Lösung des zu trennenden Substanzgemisches mit einer chiralen stationären Phase, beinhaltend ein Trägermaterial und einen chiralen Selektor gemäß Struktur (II) oder (IIa), (ii) die Auftrennung der Bestandteile des Substanzgemisches aufgrund ihrer unterschiedlichen Wechselwirkung mit der stationären Phase unter Verwendung einer mobilen Phase und (iii) gegebenenfalls das fraktionierende Sammeln der mobilen Phase und die Isolierung der chromatographisch gereinigten Substanz hieraus. Dieses Verfahren eignet sich darüber hinaus zur Minorspurenbestimmung von Enantiomeren, bevorzugt von Enantiomeren von α- und β-Aminosäuren. Bevorzugt sind flüssigchromatographische Verfahren, insbesondere HPLC-Verfahren. Diese können beispielsweise im Normalphasenmodus (NP), Polar-Organischen (PO) oder Umkehrphasenmodus (RP) ohne Zusatz von Metallionen durchgeführt werden.

Des weiteren stellt die vorliegende Erfindung eine chirale stationäre Phase zur chromatographischen Trennung von Substanzgemischen, bevorzugt von Gemischen chiraler Substanzen, bevorzugt von Enantiomerengemischen, insbesondere von Enantiomeren von Substanzen ausgewählt aus der Gruppe enthaltend β-Aminosäuren und ihre Derivate, α-Aminosäuren und α-Hydroxysäuren, zur Verfügung, beinhaltend ein Trägermaterial und einen chiralen Selektor, wobei der chirale Selektor ein in α-Position unsubstituiertes β-Aminosäurederivat der Struktur (III) 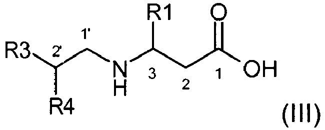 darstellt, worin R¹ = (C₁-C₄)-Alkyl, (C₆-C₁₀)-Aryl, (C₇-C₁₃)-Aralkyl, (C₇-C₁₀)-Heteroaralkyl, Pyridyl, Hydroxymethyl, CH(OH)CH₃, CH₂CONH₂, CH₂COOH, (CH₂)₂CONH₂, (CH₂)₂COOH, (CH₂)₄-NH₂, (CH₂)₂-SCH₃oder (CH₂)₃NHC(NH)NH₂ und R³ = H oder OH und R⁴ = (C₁-C₂₀)-Alkyl, (C₆-C₁₀)-Aryl oder (C₇-C₁₃)-Aralkyl bedeutet.

Erfindungsgemäß liegt der chirale Selektor überwiegend in einer absoluten Konfiguration bezüglich des Kohlenstoffatomes C3 (β-Kohlenstoffatom) vor, während das in 2'-Position potentiell vorhandene Stereozentrum nicht in überwiegend einer absoluten Konfiguration vorliegen muss.

Bevorzugt bedeutet R¹ = Phenyl.

R⁴ stellt im Sinne der Erfindung bevorzugt einen kurzkettig substituierten Phenylrest, besonders bevorzugt einen unsubstituierten Phenylrest, oder einen linearen oder verzweigten Alkylrest mit 1 bis 20 Kohlenstoffatomen, bevorzugt mit 4 bis 18 Kohlenstoffatomen, besonders bevorzugt mit 6 bis 16 Kohlenstoffatomen, insbesondere mit 10 bis 12 Kohlenstoffatomen und besonders bevorzugt n-Decyl dar. Insbesondere ist die Ausführungsform bevorzugt, in der R¹ = Phenyl und R⁴ = n-Decyl bedeutet.

Die Aufbringung der chiralen Selektoren gemäß Formel (III) auf geeignete Trägermaterialien erfolgt bevorzugt adsorptiv durch sogenannte dynamische Belegung an Umkehrphasenmaterialien (Reversed-Phase-Materialien, RP-Materialien), bevorzugt auf Kieselgel- oder Monolithbasis. Bevorzugte RP-Materialien sind z.B. RP-2, RP-4, RP-5, RP-6, RP-8, RP-12 und insbesondere RP-18.

Die Erfindung stellt weiterhin ein Verfahren zur Herstellung einer chiralen stationären Phase enthaltend einen chiralen Selektor gemäß Struktur (III) bereit, umfassend die folgenden Schritte: (i) Darstellung der *N*-alkylierten Aminosäure, (ii) adsorptive Anbindung der *N*-alkylierten Aminosäure an ein Trägermaterial.

Die Herstellung der Verbindungen der allgemeinen Struktur (III) kann beispielsweise durch Umsetzung einer entsprechenden Epoxidverbindung mit der jeweiligen β-Aminosäure unter Einsatz äquimolarer Mengen starker Base, z.B. Natriummethylat, erfolgen (Busker et al., DE 3 143 726 A1). Dies führt zur racemischen Konfiguration am 2'-Kohlenstoffatom. Eine alternative Methode zur Darstellung chiraler Selektoren der Struktur (III) ist die *N*-Alkylierung der entsprechenden β-Aminosäure mit einem n-Alkylbromid (z. B. gemäß Davankov et al., Chromatographia 1980, 13, 677-685).

Bevorzugt ist weiterhin ein Verfahren zur chromatographischen Trennung von Substanzgemischen, bevorzugt von Gemischen chiraler Substanzen, besonders bevorzugt von Enantiomerengemischen, insbesondere von Enantiomeren von Substanzen ausgewählt aus der Gruppe enthaltend β-Aminosäuren und ihre Derivate, α-Aminosäuren und α-Hydroxysäuren, im analytischen, semipräparativen oder präparativen Maßstab unter Verwendung der durch Struktur (III) beschriebenen chiralen Selektoren oder der auf ihnen basierenden chiralen stationären Phasen, insbesondere im Ligandenaustauschmodus (LE) unter Zusatz von Metallionen, bevorzugt zweiwertigen Metallionen, und besonders bevorzugt Kupfer(II)ionen. Ein solches Verfahren beinhaltet (i) das In-Kontakt-Bringen einer Lösung des zu trennenden Substanzgemisches mit einer chiralen stationären Phase beinhaltend einen chiralen Selektor gemäß Struktur (III), (ii) Auftrennung der einzelnen Bestandteile des Substanzgemisches aufgrund ihrer unterschiedlichen Wechselwirkung mit dem chiralen Selektor unter Verwendung einer mobilen Phase enthaltend zweiwertige Übergangsmetallionen, bevorzugt Kupfer(II)ionen und (iii) im Falle einer präparativen oder semipräparativen flüssigchromatographischen Anwendung gegebenenfalls das fraktionierende Sammeln der mobilen Phasen und die Isolierung der chromatographisch gereinigten Substanz hieraus. Dieses Verfahren eignet sich darüber hinaus zur Minorspurenbestimmung von Enantiomeren, bevorzugt von Enantiomeren von α- und β-Aminosäuren.

Chromatographische Trennung bezeichnet im Sinne der Erfindung die Auftrennung von Substanzgemischen, bevorzugt von Gemischen chiraler Substanzen, besonders bevorzugt von Enantiomerengemischen, insbesondere von Enantiomeren von Substanzen ausgewählt aus der Gruppe enthaltend β-Aminosäuren und ihre Derivate, α-Aminosäuren und α-Hydroxysäuren, an einer stationären Phase, sowohl zu analytischen Zwecken ohne nachfolgende Isolierung der getrennten Substanzen als auch zu semipräparativen oder präparativen Zwecken mit nachfolgender Isolierung der getrennten Substanzen von Bestandteilen des chromatographischen Systems (Trägermaterial, mobile Phase) und anderen in der Probe enthaltenen Substanzen.

In überwiegend einer absoluten Konfiguration bedeutet im Sinne der Erfindung mehr als 50%, bevorzugt mindestens 90 %, insbesondere mindestens 95 % einer optisch aktiven enantiomeren Form, und besonders bevorzugt die enantiomerenreine Form.

### Beschreibung der Abbildungen

Figur 1 zeigt die chromatographische Auflösung optischer Isomere unterschiedlicher β-Aminosäurederivate unter Verwendung chiraler stationärer Phasen gemäß Beispiel 1.
Figur 2 zeigt die chromatographische Auflösung optischer Isomere unterschiedlicher β-Aminosäurederivate unter Verwendung chiraler stationärer Phasen gemäß Beispiel 2.
Figur 3 zeigt die chromatographische Auflösung optischer Isomere unterschiedlicher α- und β-Aminosäuren sowie α-Hydroxysäuren unter Verwendung chiraler stationärer Phasen gemäß Beispiel 3.

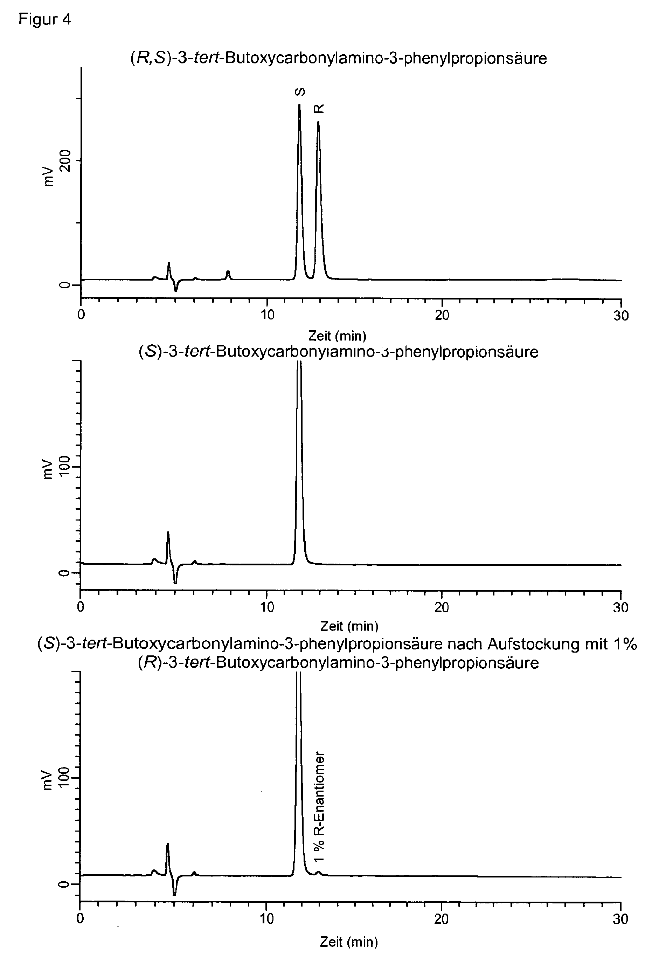

Figur 4 zeigt die Anwendung einer chiralen stationären Phase gemäß Beispiel 2 zur Bestimmung der Enantiomerenreinheit, insbesondere im Bereich der Minorspurenbestimmung. Das obere Chromatogramm zeigt die chromatographische Auflösung eines racemischen Gemisches von (*R*)- und (S)-3-*tert-*Butoxycarbonylamino-3-phenylpropionsäure unter Verwendung einer chiralen stationären Phase gemäß Beispiel 2. In der Mitte ist das Chromatogramm der reinen (*S*)-3-*tert*-Butoxycarbonylamino-3-phenylpropionsäure an dieser Phase dargestellt. Das untere Chromatogramm zeigt die Auflösung von (*S*)-3-*tert*-Butoxycarbonylamino-3-phenylpropionsäure, die mit 1 % (*R*)-3-*tert*-Butoxycarbonylamino-3-phenylpropionsäure aufgestockt wurde.

Aus dem Stand der Technik ist wie bereits erwähnt eine Vielzahl chiraler Selektoren und auf diesen basierender stationärer Phasen bekannt. So fanden beispielsweise zur Trennung von β-Aminosäuren und ihren Derivaten mittels Hochleistungsflüssigchromatographie bisher chirale Selektoren auf der Basis von Kronenether, α-Aminosäurederivaten oder makrozyklischen Glycopeptiden Anwendung. In Tabelle 1 sind verschiedene chromatographische Verfahren zur Trennung enantiomerer β-Aminosäuren, die aus dem Stand der Technik bekannt sind, zusammengefasst und Ergebnissen, die mit der Lehre der vorliegenden Erfindung erzielt werden können, gegenübergestellt. Die Charakterisierung der Trennsäulen erfolgt in üblicher Weise - wie z.B. in Meyer, Praxis der Hochdruckflüssigkeitschromatographie, Wiley-VCH Weinheim 2004, beschrieben - durch die Kapazitätsfaktoren k₁ und k₂, den Trennfaktor α und die Auflösung Rₛ. Die genauen chromatographischen Bedingungen sind der jeweiligen Originalliteratur zu entnehmen.

**Tabelle 1: Chromatographische Trennung von Enantiomerengemischen von β-Aminosäuren der allgemeinen Struktur H₂N-CHR¹-CH₂-CO₂H an chiralen stationären Phasen**

| **R¹** | **Chiraler Selektor** | **Mobile Phase*** | **k₁** | **α** | **Rₛ** | **Ref.** |
|---|---|---|---|---|---|---|
| **Ph** | Vancomycin | MeOH/AcOH/TFA 100:0.1:0.1 | 0.70 | 1.13 | 0.80 | [1] |
| | (*S*)-Leucinol | MeOH/H₂O 50:50 + CuSO₄ (0.3 mM) | 1.38 | 1.61 | 1.48 | [2] |
| | Ristocetin A | MeOH/AcOH/TFA 100:0.4:0.1 | 2.61 | 1.25 | 1.54 | [3] |
| | A-40,926 | MeOH/H₂O 90:10 + NH₄OAc (25 mM) | 0.71 | 1.33 | 2.03 | [4] |
| | Teicoplanin | MeOH/H₂O 90:10 + NH₄OAc (25 mM) | 0.73 | 1.32 | 2.05 | [4] |
| | (18-Krone-6)-2,3,11,12-tetracarbonsäure | MeOH/H₂O 50:50 + AcOH (5 mM) | 3.60 | 1.60 | 2.76 | [5] |
| | (*R*)-Phenylglycinol | MeOH/H₂O 50:50 + CuSO₄ (0.3 mM) | 4.41 | 2.03 | 4.15 | [2] |
| | **3-Amino-3-phenylpropionsäure** | MeOH/H₂O 90:10 + Cu(OAc)₂ (0.1 mM) | **3.16** | **1.72** | **5.08** | ^{a} |
| **3-MeOC₆H₄** | Teicoplanin | MeOH/AcOH/TFA 100:0.01:0.01 | 2.66 | 1.00 | 0.0 | [1] |
| | Vancomycin | MeOH/AcOH/TFA 100:0.1:0.1 | 0.75 | 1.16 | 0.80 | [1] |
| | Ristocetin A | MeOH/AcOH/TFA 100:0.1:0.1 | 1.43 | 1.15 | 1.00 | [1] |
| | (18-Krone-6)-2,3,11,12-tetracarbonsäure | MeOH/H₂O 50:50 + AcOH (5 mM) | 3.83 | 1.31 | 1.71 | [6] |
| | 4-(3,5-Dinitrobenzamido)-1,2,3,4-tetrahydrophenanthren | n-Hexan/EtOH/TFA 90:10:0.2 | n.a.^{b} | 2.6 | 2.1 | [7] |
| | **3-Amino-3-phenylpropionsäure** | MeOH/H₂O 90:10 + Cu(OAc)₂ (0.1 mM) | **6.72** | **1.63** | **5.13** | ^{a} |
| **2-ClC₆H₄** | Teicoplanin | MeOH/AcOH/TFA 100:0.01:0.01 | 1.46 | 1.08 | <0.40 | [1] |
| | Ristocetin A | MeOH/AcOH/TFA 100:0.1:0.1 | 1.23 | 1.11 | 0.70 | [1] |
| | (18-Krone-6)-2,3,11,12-tetracarbonsäure | MeOH/H₂O 20:80 + (10 mM AcOH) | 3.04 | 1.14 | 1.06 | [6] |
| | **3-Amino-3-phenylpropionsäure** | MeOH/H₂O 90:10 + Cu(OAc)₂ (0.1 mM) | **10.88** | **3.34** | **10.6** | ^{a} |

| | | | | | | |
|---|---|---|---|---|---|---|
| ^{a} vorliegende Erfindung; ^{b} n. a. = nicht angegeben * weitere Parameter: vgl. Literatur / Beispiele dieser Erfindung: Fluss 1 ml/min, 30 °C [1] Sztojkov-Ivanov et al., Chromatographia 2006, 64, 89-94. [2] Hyun et al., J. Sep. Sci. 2003, 26, 1615-1622. [3] Peter et al., J. Chromatogr. A 2001, 926, 229-238. [4] D'Acquarica et al., Tetrahedron: Asymmetry 2000, 11, 2375-2385. [5] Hyun et al., J. Sep. Sci. 2002, 25, 648-652. [6] Berkecz et al., J. Chromatogr. A 2006, 1125, 138-143. [7] Madhavan, Chromatographia 2007, 66, 243-246. | | | | | | |

Wie aus Tabelle 1 ersichtlich ist, erweisen sich die erfindungsgemäßen Selektoren und die auf ihnen basierenden stationären Phasen als äußerst vorteilhaft gegenüber den aus dem Stand der Technik bekannten Selektoren bezüglich ihrer Trennleistung, charakterisiert durch den Trennfaktor α und die Auflösung der Enantiomere Rₛ, gerade auch im Hinblick auf semipräparative und präparative Anwendungen. Darüber hinaus zeigen sie eine breite Anwendbarkeit; hier sei auch auf die in Tabellen 2-4 dargestellten Ergebnisse verwiesen.

Aus dem Stand der Technik sind weiterhin chirale Selektoren bekannt, die auf α-Aminosäuren basieren und eine gewisse Ähnlichkeit zu den durch Struktur (II) beschriebenen Selektoren aufweisen (Welch, J. Chromatogr. A 1994, 3-26). Bei keinem der dort beschriebenen chiralen Selektoren handelt es sich jedoch um ein in α-Position unsubstituiertes β-Aminosäurederivat. Es wird im Stand der Technik sogar in diversen Quellen beschrieben, dass sich eine größere Distanz zwischen den für die chirale Erkennung relevanten funktionellen Gruppen und eine höhere konformationelle Flexibilität des Selektors, die sich aufgrund der zusätzlichen Methylengruppe bei den erfindungsgemäßen Selektoren gegenüber α-Aminosäure, oder auch gegenüber in α-Position substituierten β-Aminosäuren ergeben, negativ auf die Trennleistung auswirken (siehe u. a. Pirkle und McCune, J. Chromatogr. 1988, 441, 311; Wang et al., Anal. Chem. 2000, 72, 5459-5465; Welch, J. Chromatogr. A 1994, 3-26).

### Beispiele

Die nachfolgenden Beispiele erläutern die Erfindung näher, schränken sie jedoch keinesfalls ein.

### Beispiel 1

Herstellung einer chiralen stationären Phase unter kovalenter Anbindung von (*S*)-3-(3,5-Dinitrobenzamido)-3-phenylpropionsäure an eine aminopropylfunktionalisierte Kieselgelmatrix (Aminophase)

### (1) Synthese von (S)-3-(3,5-Dinitrobenzamido)-3-phenylpropionsäure

16 g (*S*)-3-Amino-3-phenylpropionsäure ((*S*)-ß-Phenylalanin) werden mit 80 ml 5%iger wässriger Natronlauge versetzt, und die resultierende Lösung wird durch Zugabe von 50%iger wässriger Natronlauge auf einen pH-Wert von 11-12 eingestellt. Nach Zugabe von 20 ml Tetrahydrofuran wird die Reaktionsmischung auf 10 °C abgekühlt, eine Lösung von 23.1 g Dinitrobenzoylchlorid in 60 ml Tetrahydrofuran wird über einen Zeitraum von 60 min hinzugegeben, und die Reaktionsmischung wird weitere 60 min gerührt. Die organische Phase wird dann destillativ entfernt, und die verbleibende wässrige Phase wird zweimal mit Methyl-tert-butylether extrahiert. Die wässrige Phase mit 200 ml Ethylacetat versetzt und durch Zugabe von 4 N Salzsäure auf einen pH-Wert von 1 - 2 eingestellt. Die Phasen werden getrennt, und die organischen Phase wird mit 200 ml gesättigter Kochsalzlösung gewaschen. Nach Abdestillieren des Lösungsmittels bei 45 °C und p = 150 mbar wird der Rückstand in 300 ml Cyclohexan aufgenommen. Das Produkt wird abfiltriert und bei 40 °C getrocknet. Die weitere Reinigung des Produktes erfolgt mittels präparativer Hochleistungsflüssigkeitschromatographie an einem Reversed-Phase-Material (z.B. Kromasil RP-18, Korngröße 10 µm) im Lösungsmittelgemisch Acetonitril/Wasser/Trifluoressigsäure. Ausbeute: 8.0 g, HPLC-Reinheit 99.8 FI.-%; Elementaranalyse: 53.4 % C, 3.6 % H, 12.7 % N; ¹H-NMR (DMSO) δ (ppm): 2.85 (dd, 1 H), 2.96 (dd, 1 H), 5.48 (m, 1 H), 7.26 (t, 1 H), 7.35 (t, 2H), 7.44 (d, 2H), 8.96 (t, 1 H), 9.07 (d, 2H), 9.58 (d, 1 H), 13.30 (s, 1 H, CO₂*H*).

### (2) Anbindung von (S)-3-(3,5-Dinitrobenzamido)-3-phenylpropionsäure an eine aminopropylfunktionalisierte Kieselgelphase (Aminophase)

In einer Lösung von 4 g (*S*)-3-(3,5-Dinitrobenzamido)-3-phenylpropionsäure in 200 ml Tetrahydrofuran werden 4.1 g Aminophase (YMC-Gel Amino NH12S05, Korngröße 5 µm) suspendiert, und das Reaktionsgemisch wird unter Rühren mit 3.2 g *N-*Ethoxycarbonyl-2-ethoxy-1,2-dihydrochinolin (EEDQ) versetzt. Nach 8 h bei Raumtemperatur wird das Phasenmaterial abfiltriert, jeweils mit 50 ml Methanol und Diethylether gewaschen und getrocknet. Ausbeute: 4.6 g des gewünschten Trägermaterials, Elementaranalyse: 10.6 % C, 1.2 % H und 2.4 % N; entsprechend einer Belegung von 0.4 mmol/g.

Das Befüllen einer 250 x 5 mm HPLC Säule erfolgt bei 300 bar nach der Balanceddensity-Methode (siehe Meyer, Praxis der Hochdruckflüssigkeitschromatographie, Wiley-VCH: Weinheim 2004).

### Anwendung Beispiel 1

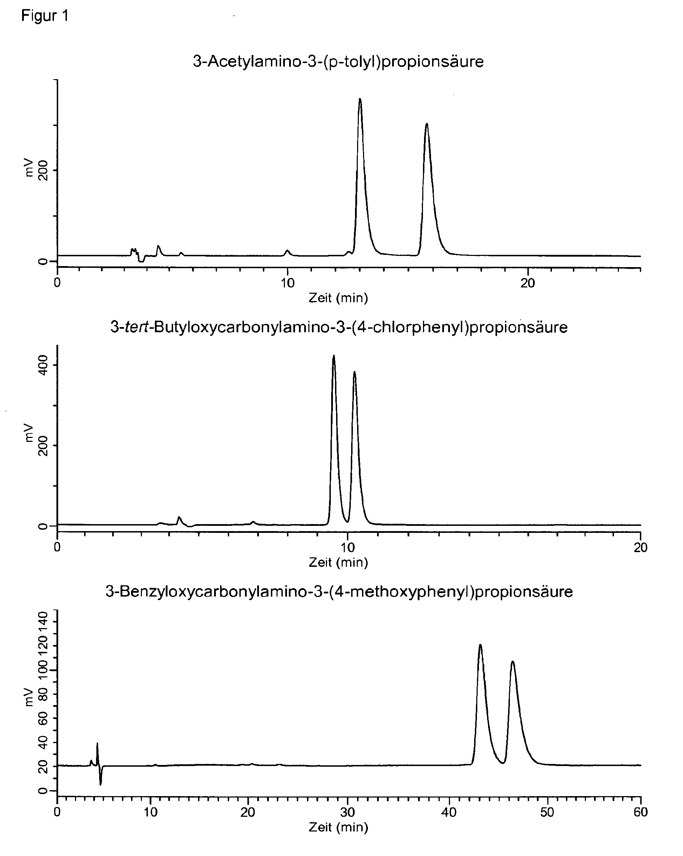

Die gemäß Beispiel 1 hergestellten Trennsäulen sind unter HPLC-Bedingungen zur Chromatographie einer Vielzahl von β-Aminosäuren bzw. β-Aminosäurederivaten geeignet (Tabelle 2). Figur 1 zeigt die chromatographische Auflösung optischer Isomere unterschiedlicher β-Aminosäurederivate unter Verwendung von Trennsäulen gemäß Beispiel 1.

**Tabelle 2**

| Substanz | Mobile Phase* | k'₁ | k'₂ | α | Rₛ |
|---|---|---|---|---|---|
| 3-Acetylamino-3-(4-fluorphenyl)-propionsäure | A | 2.30 | 2.65 | 1.15 | 2.21 |
| 3-Acetylamino-3-(4-chlorphenyl)-propionsäure | A | 2.39 | 3.01 | 1.26 | 3.79 |
| 3-Acetylamino-3-(p-tolyl)propionsäure | A | 2.73 | 3.51 | 1.29 | 4.24 |
| 3-Acetylamino-3-(4-methoxyphenyl)propionsäure | A | 4.71 | 6.41 | 1.36 | 5.52 |
| 3-Acetylamino-3-(4-nitrophenyl)-propionsäure | A | 4.59 | 5.00 | 1.09 | 1.30 |
| 3-Acetylamino-3-phenylpropionsäure | A | 3.12 | 3.71 | 1.19 | 2.77 |
| 3-*tert*-Butyloxycarbonylamino-3-(4-fluorphenyl)propionsäure | B | 1.69 | 1.83 | 1.09 | 1.35 |
| 3-*tert*-Butyloxycarbonylamino-3-(4-methoxyphenyl)propionsäure | B | 3.57 | 4.41 | 1.23 | 4.24 |
| 3-*tert*-Butyloxycarbonylamino-3-(p-tolyl)propionsäure | B | 2.07 | 2.46 | 1.19 | 3.19 |
| 3-*tert*-Butyloxycarbonylamino-3-(4-chlorphenyl)propionsäure | B | 1.73 | 1.93 | 1.11 | 1.85 |
| 3-*tert*-Butyloxycarbonylamino-3-(4-nitrophenyl)propionsäure | B | 3.50 | 3.70 | 1.06 | 1.08 |
| 3-*tert*-Butyloxycarbonylamino-3-phenylpropionsäure | B | 2.15 | 2.41 | 1.12 | 2.01 |
| 3-Benzyloxycarbonylamino-3-(p-tolyl)propionsäure | B | 6.33 | 6.67 | 1.05 | 1.13 |
| 3-Benzyloxycarbonylamino-3-(4-methoxyphenyl)propionsäure | B | 11.32 | 12.26 | 1.08 | 1.75 |

| | | | | | |
|---|---|---|---|---|---|
| Mobile Phase A: Isohexan / Ethanol / Trifluoressigsäure (800+200+1, v/v/v) Mobile Phase B: Isohexan / Methyl-*tert*-butylether / Ethanol / Trifluoressigsäure (800+150+ 50+1, v/v/v/v) * weitere Parameter: Fluss 1 ml/min, Temperatur 30 °C | | | | | |

### Beispiel 2

### Herstellung einer chiralen stationären Phase unter kovalenter Anbindung von (S)-3-[(S)-3-(3,5-Dinitrobenzamido)-3-phenylpropanamido]-3-phenylpropionsäure (3-(3,5-Dinitrobenzoyl)-(S)-ß-phenylalanyl-(S)-ß-phenylalanin) an eine aminopropylfunktionalisierte Kieselgelmatrix (Aminophase)

### (1) Synthese von (S)-3-[(S)-3-(3,5-Dinitrobenzoyl)-3-phenylpropanamido]-3-phenylpropionsäure (3-(3,5-Dinitrobenzoyl)-(S)-β-phenylalanyl-(S)-ß-phenylalanin)

### a. Synthese von (S)-3-(3,5-Dinitrobenzamido)-3-phenylpropionsäureethylester

11.49 g (*S*)-3-Amino-3-Phenylpropionsäureethylester Hydrochlorid werden in 100 ml Tetrahydrofuran suspendiert und im Eisbad auf 0 - 10 °C abgekühlt. Nach Zugabe von 14 ml Triethylamin wird unter Rühren und Kühlung über einen Zeitraum von 30 min eine Lösung von 11.52 g 3,5-Dinitrobenzoylchlorid in 40 ml Tetrahydrofuran hinzugegeben. Das Kühlbad wird entfernt, und das Reaktionsgemisch wird weitere 120 min gerührt, wobei es sich auf Raumtemperatur erwärmen kann. Das Reaktionsgemisch wird filtriert, und das Filtrat wird zur Trockene eingeengt (Produktfraktion 1). Der Filterkuchen - bestehend aus Triethylamin Hydrochlorid und weiterem Produkt - wird in 100 ml Ethylacetat aufgenommen und 20 min bei 35 °C digeriert. Die Suspension wird filtriert, und der Filterkuchen wird verworfen. Das Filtrat wird bei 40 °C unter vermindertem Druck zur Trockene eingeengt (Produktfraktion 2). Die Produktfraktionen werden vereinigt. Ausbeute 16.4 g; HPLC-Reinheit > 97 FI.%. Zur weiteren Reinigung wird das Rohprodukt in 400 ml Wasser 2 h bei Raumtemperatur digeriert und anschließend filtriert. Die Feuchtausbeute beträgt 15.4 g; HPLC-Reinheit 99,8 FI.-%. Das Produkt wird ohne weitere Reinigung weiter umgesetzt.

### b. Verseifung zur (S)-3-(3,5-Dinitrobenzamido)-3-phenylpropionsäure

15.4 g (*S*)-3-(3,5-Dinitrobenzamido)-3-phenylpropionsäureethylester aus Reaktion a werden in 100 ml Tetrahydrofuran gelöst, und die resultierende Lösung wird mit 50 ml Wasser versetzt. Unter starkem Rühren wird das schwach trübe Reaktionsgemisch auf 45 °C erwärmt, und der pH-Wert wird mit 32 %iger wässriger Natronlauge auf 13 bis 13.5 eingestellt und für den Verlauf der Reaktion konstant gehalten. Nach Beendigung der Reaktion (HPLC-Kontrolle > 99 FI.-% Umsatz) wird der pH-Wert der Lösung durch Zugabe von 6 N Salzsäure auf 7 - 8 eingestellt, anschließend wird das organische Lösungsmittel unter vermindertem Druck abdestilliert. Die wässrige Lösung wird mit Wasser auf 350 ml verdünnt, und unter starkem Rühren wird der pH-Wert mit 6 N Salzsäure auf 1 - 2 eingestellt. Das ausgefallene Produkt wird abgesaugt und zweimal mit Wasser gewaschen. Nach Trocknen bei 40 °C unter vermindertem Druck wird eine Ausbeute von 13.67 g ermittelt. Die HPLC-Reinheit beträgt 99,8 FI.-%.

### c. Synthese von (S)-3-(3,5-Dinitrobenzamido)-3-phenylpropionsäure(2,5-dioxopyrrolidin-1-yl)ester (3-(3,5-Dinitrobenzoyl)-(S)-β-phenylalanin(2,5-dioxopyrrolidin-1-yl)ester)

12 g (*S*)-3-(3,5-Dinitrobenzamido)-3-phenylpropionsäure aus Reaktion b) werden in 120 ml Tetrahydrofuran gelöst und mit 3.92 g *N*-Hydroxysuccinimid versetzt. Unter Kühlung im Eisbad werden bei 0 - 5 °C portionsweise 7.1 g Dicyclohexylcarbodümid zur Reaktionsmischung hinzugegeben. Nach vollständiger Zugabe lässt man den Ansatz unter weiterem Rühren im Eisbad stehen, bis die Lösung Raumtemperatur erreicht. Nach Beendigung der Reaktion (HPLC-Kontrolle > 95 FI.-% Umsatz) wird der ausgefallene Dicyclohexylharnstoff abfiltriert und mit Tetrahydrofuran gewaschen. Das Fitrat wird unter vermindertem Druck konzentriert, und der Rückstand wird in 250 ml 2-Propanol 2 h am Rückfluß digeriert. Das Produkt wird abgesaugt, mit 2-Propanol gewaschen und bei 50 °C unter vermindertem Druck getrocknet. Die Ausbeute beträgt 13.87 g; HPLC-Reinheit 98,8 FI.-%.

### d. Umsetzung zur (S)-3-[(S)-3-(3,5-Dinitrobenzamido)-3-phenylpropanamido]-3-phenylpropionsäure (3-(3,5-Dinitrobenzoyl)-(S)-ß-phenylalanyl-(S)-ß-phenylalanin)

5.65 g (*S*)-3-Amino-3-phenylpropionsäure werden in 70 ml Wasser suspendiert, der pH-Wert der Suspension wird durch Zugabe von Natronlauge auf 10.5 bis 11 eingestellt, und 70 ml Tetrahydrofuran werden hinzugegeben. Der Ansatz wird im Eisbad auf 0 - 5 °C gekühlt und unter Rühren werden 13 g (*S*)-3-(3,5-Dinitrobenzamido)-3-phenylpropionsäure(2,5-dioxopyrrolidin-1-yl)ester portionsweise über einen Zeitraum von 15 min zugegeben. Nach vollständiger Zugabe wird der pH-Wert des Reaktionsgemisches durch Zugabe von Natronlauge zwischen 9 - 9.5 gehalten. Nach Erreichen eines konstanten pH-Wertes bei nahezu vollständigem Umsatz (HPLC-Kontrolle; < 0,5 FI.-% Edukt) wird die Lösung mit Wasser auf ein Volumen von etwa 800 ml verdünnt, durch Zugabe von 4 N Salzsäure wird ein pH-Wert von 1.5 - 2 eingestellt, und es wird weitere 30 min gerührt. Der Feststoff wird abgesaugt, mit Wasser gewaschen und bei 50 °C unter vermindertem Druck getrocknet. Der getrocknete Feststoff wird in 250 ml Tetrahydrofuran 20 min bei 40 °C digeriert, die Suspension wird abgekühlt, und der Feststoff (Produktfraktion 1) wird abgesaugt. Die Mutterlauge wird bei 40 °C Wasserbadtemperatur auf ein Volumen von etwa 70-80 ml konzentriert, die entstehende Suspension wird auf Raumtemperatur abgekühlt, und der Feststoff (Produktfraktion 2) wird abgesaugt. Die Produktfraktionen werden vereinigt und unter vermindertem Druck bei 50 °C getrocknet. Die Ausbeute beträgt 11.1 g; HPLC-Reinheit > 99,5 FI.-%; ¹H-NMR (DMSO) δ (ppm): 2.64 (d, 2H), 2.77 (m, 2H), 5.15 (m, 1 H), 5.52 (m, 1 H), 7.10-7.20 (m, 5H), 7.25 (t, 1 H), 7.31 (t, 2H), 7.39 (d, 2H), 8.44 (d, 1 H), 8.95 (m, 1 H), 9.01 (m, 2H), 9.53 (d, 1 H), 12.17 (s, 1 H, CO₂H).

### (2) Anbindung der (S)-3-[(S)-3-(3,5-Dinitrobenzamido)-3-phenylpropanamido]-3-phenylpropionsäure an eine aminopropylfunktionalisierte Kieselgelphase (Aminophase)

In einer Lösung von 4.7 g (*S*)-3-[(S)-3-(3,5-Dinitrobenzamido)-3-phenylpropanamido]-3-phenylpropionsäure in 1 I Tetrahydrofuran werden 5 g Aminophase (YMC-Gel Amino NH12S05, Korngröße 5 µm) suspendiert, und die Suspension wird unter Rühren mit 4 g N-Ethoxycarbonyl-2-ethoxy-1,2-dihydrochinolin (EEDQ) versetzt. Nach 24 h bei Raumtemperatur wird das Phasenmaterial abfiltriert, mit jeweils 100 ml Methanol und Diethylether gewaschen und anschließend getrocknet. Elementaranalyse: 12.44 % C, 1.36 % H und 2.76 % N; entsprechend einer Belegung von 0.312 mmol/g.

Das Befüllen einer 250 x 5 mm HPLC Säule erfolgt bei 300 bar nach der Balanceddensity-Methode (siehe Meyer, Praxis der Hochdruckflüssigkeitschromatographie, Wiley-VCH Weinheim 2004).

### Anwendung Beispiel 2

Auch die gemäß Beispiel 2 hergestellten Trennsäulen sind unter HPLC-Bedingungen für eine Vielzahl von β-Aminosäuren bzw. β-Aminosäurederivate geeignet - teilweise mit gegenüber dem Monomeren verbesserter Selektivität. Dies wird auf eine intensivere chirale Erkennung der verschiedenen Analyten durch den dipeptidischen Selektor zurückgeführt.

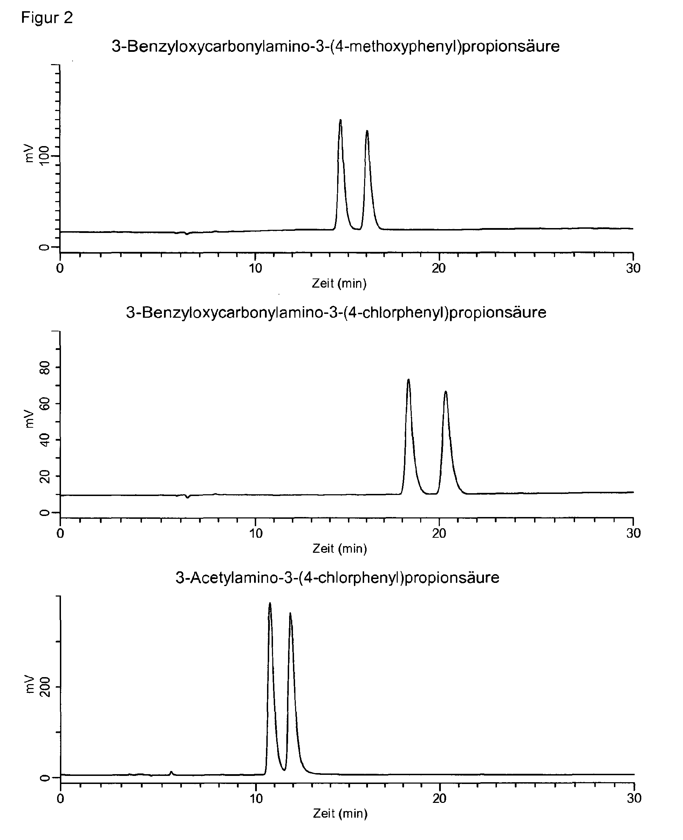

In Tabelle 3 sind die Ergebnisse zusammengefasst. Figur 2 zeigt die chromatographische Auflösung optischer Isomere von unterschiedlichen β-Aminosäure-derivaten unter Verwendung der Trennsäulen gemäß Beispiel 2.

**Tabelle 3**

| Substanz | Mobile Phase* | k'₁ | k'₂ | α | Rₛ |
|---|---|---|---|---|---|
| 3-*tert*-Butyloxycarbonylamino-3-(4-fluorphenyl)propionsäure | A | 2.26 | 2.51 | 1.11 | 1.21 |
| 3-*tert*-Butyloxycarbonylamino-3-(4-methoxyphenyl)propionsäure | A | 4.52 | 5.85 | 1.29 | 2.64 |
| 3-*tert*-Butyloxycarbonylamino-3-(p-tolyl)propionsäure | A | 2.46 | 3.02 | 1.23 | 2.35 |
| 3-*tert*-Butyloxycarbonylamino-3-(4-chlorphenyl)propionsäure | A | 2.33 | 2.72 | 1.17 | 1.74 |
| 3-*tert*-Butyloxycarbonylamino-3-(4-nitrophenyl)propionsäure | A | 5.39 | 5.97 | 1.11 | 1.03 |
| 3-Benzyloxycarbonylamino-3-(p-tolyl)propionsäure | B | 1.50 | 1.70 | 1.14 | 2.04 |
| 3-Benzyloxycarbonylamino-3-(4-methoxyphenyl)propionsäure | B | 1.52 | 1.77 | 1.17 | 2.50 |
| 3-Benzyloxycarbonylamino-3-(4-fluorphenyl)propionsäure | B | 1.61 | 1.76 | 1.11 | 1.68 |
| 3-Benzyloxycarbonylam ino-3-(4-chlorphenyl)propionsäure | B | 2.17 | 2.51 | 1.16 | 2.74 |
| 3-Benzyloxycarbonylamino-3-phenyl-propionsäure | B | 1.21 | 1.33 | 1.10 | 1.33 |
| 3-Acetylamino-3-(4-fluorphenyl)-propionsäure | C | 2.02 | 2.22 | 1.10 | 1.24 |
| 3-Acetylamino-3-(4-chlorphenyl)-propionsäure | C | 2.08 | 2.40 | 1.15 | 1.82 |
| 3-Acetylamino-3-(p-tolyl)propionsäure | C | 2.22 | 2.63 | 1.19 | 2.40 |
| 3-Acetylamino-3-(4-methoxyphenyl)-propionsäure | C | 3.76 | 4.58 | 1.22 | 2.85 |
| 3-Acetylamino-3-phenylpropionsäure | C | 2.31 | 2.62 | 1.13 | 1.66 |

| | | | | | |
|---|---|---|---|---|---|
| Mobile Phase A: Isohexan / 2-Propanol / Trifluoressigsäure (900+100+1, v/v/v) Mobile Phase B: Acetonitril / Wasser / Essigsäure (700+300+1, v/v/v) Mobile Phase C: Isohexan / Ethanol / Trifluoressigsäure (750+250+1,v/v/v) * weitere Parameter: Fluss 1 ml/min, Temperatur 30 °C. | | | | | |

### Beispiel 3

### Herstellung einer chiralen stationären Phase unter dynamischer Belegung eines RP-Trägermaterials mit (3S)-3-(2-(R,S)-Hydroxydodecylamino)-3-phenylpropionsäure

### (1) Synthese von (3S)-3-(2-(R,S)-Hydroxydodecylamino)-3-phenylpropionsäure

In 800 ml Methanol werden 27.01 g Natriummethylat und 82.6 g (*S*)-3-Amino-3-phenylpropionsäure gelöst. Nach Zugabe von 92 g 1,2-Epoxydodecan wird die Lösung 20 h bei Raumtemperatur gerührt. Anschließend wird mit methanolischer Salzsäure der pH-Wert der Lösung auf pH 6 gestellt und das auskristallisierende Natriumchlorid abfiltriert. Nach Abdestillieren des Methanols verbleibt ein öliger Rückstand, der beim Verrühren mit 700 ml Aceton kristallisiert. Es resultieren 153.4 g farblose Kristalle. ¹H-NMR (DMSO) δ (ppm): 0.85 (t, 3H), 1.13-1.31 (m, 18H), 2,28/2,60/2,70/2.86 (m, 2H), 3.02 (m, 1H), 3.34 (m, 1H), 3.66-3.78 (m, 1H), 4.57 (m, 1H), 5,20/5,29 (s, br., 1H, O*H*), 7.42 (m, 3H), 7.60 (m, 2H), 9.40 (s, br., 2H,N*H*₂⁺), 12,56 (s, 1H, CO₂*H*)

### (2) Dynamische Belegung eines RP18-Trägermaterials

1 g (3*S*)-3-(2-(*R,S*)-Hydroxydodecylamino)-3-phenylpropionsäure werden in 50 ml Methanol gelöst (Selektorlösung), und die zu belegende stationäre Phase (Kromasil C18, Säulenlänge 250 mm, Säuleninnendurchmesser 4,6 mm) wird mit Methanol gespült. Anschließend wird die Selektorlösung mit einem Fluss von 1 mL/min 3 h rezyklisierend über die Säule gepumpt. Nach einem methanolischen Spülschritt bei einer Flussrate von 4 mL/min wird eine gesättigte methanolische Kupfer(II)acetatlösung etwa 30 min bei Raumtemperatur und einem Fluss von 1 mL/min über die Säule gepumpt. Danach ist die Trennsäule für den chromatographischen Einsatz vorbereitet.

### Anwendung Beispiel 3

Die gemäß Beispiel 3 modifizierten Trennsäulen sind im Ligandenaustauschmodus zur Enantiomerentrennung für eine Vielzahl von β-Aminosäuren und ihren Derivaten, aber auch α-Aminosäuren und α-Hydroxysäuren geeignet.

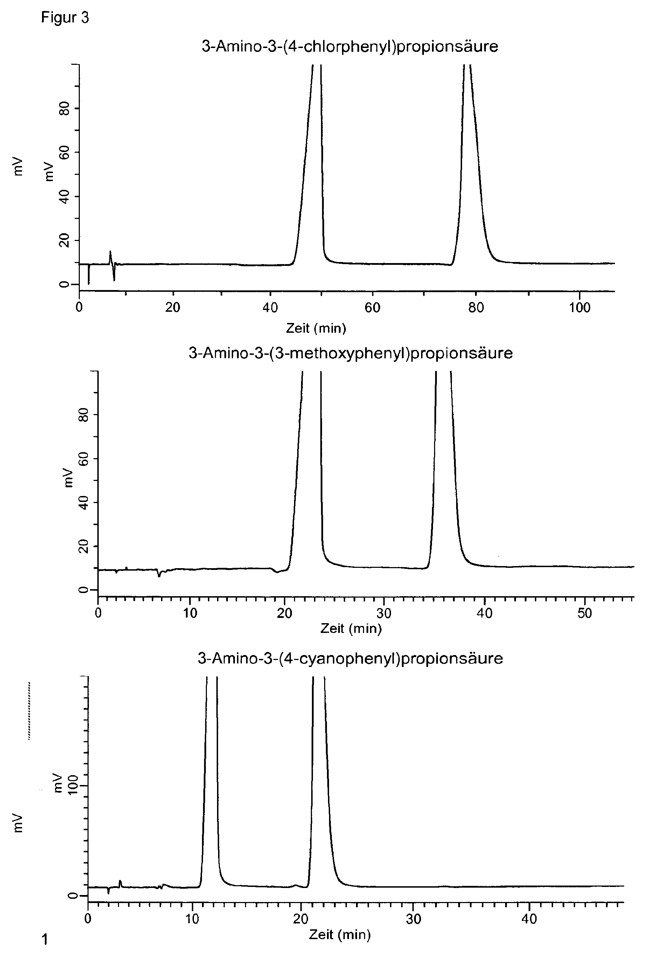

In Tabelle 4 sind die Ergebnisse zusammengefasst. Figur 3 zeigt die chromatographische Auflösung optischer Isomere unterschiedlicher β-Aminosäuren unter Verwendung der Trennsäulen gemäß Beispiel 3.

**Tabelle 4**

| Substanz | k'₁ | k'₂ | α | Rₛ |
|---|---|---|---|---|
| 3-Amino-3-(4-chlorphenyl)-propionsäure | 15.43 | 25.10 | 1.63 | 5.44 |
| 3-Amino-3-(3-methoxyphenyl)-propionsäure | 6.72 | 10.95 | 1.63 | 5.13 |
| 3-Amino-(4-cyanophenyl)-propionsäure | 3.04 | 6.13 | 2.02 | 6.03 |
| 3-Amino-3-thiophen-2-yl-propionsäure | 2.37 | 4.10 | 1.73 | 3.38 |
| 3-Amino-3-(2-chlorphenyl)-propionsäure | 10.88 | 36.3 | 3.34 | 10.6 |
| 3-Amino-3-(3-chlorphenyl)-propionsäure | 15.41 | 24.50 | 1.59 | 4.94 |
| 2-Hydroxy-2-phenylessigsäure | 10.81 | 13.81 | 1.28 | 1.26 |
| 2-Amino-3-phenylpropionsäure | 14.61 | 26.92 | 1.84 | 2.75 |
| 3-Amino-3-phenylpropionsäure | 3.16 | 5.44 | 1.72 | 5.08 |
| 3-Amino-3-(4-fluorphenyl)-propionsäure | 4.14 | 6.54 | 1.58 | 5.57 |
| 3-Amino-3-(3-fluorphenyl)-propionsäure | 4.87 | 7.41 | 1.52 | 4.10 |

| | | | | |
|---|---|---|---|---|
| Mobile Phase: 0.1 mM Kupfer(II)acetatlösung / Methanol (900 +100, v/v),Fluss: 1 ml/min, Temperatur: 30 °C. | | | | |

## Patentansprüche

1. Verwendung von in α-Position unsubstituierten β-Aminosäurederivaten der Struktur 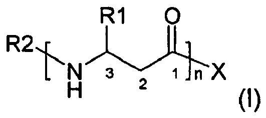 als chirale Selektoren in Verfahren zur Trennung von Substanzgemischen, bevorzugt von Gemischen chiraler Substanzen, besonders bevorzugt von Enantiomeren, insbesondere von Enantiomeren von Substanzen ausgewählt aus der Gruppe enthaltend β-Aminosäuren und ihre Derivate, α-Aminosäuren und α-Hydroxysäuren, **dadurch gekennzeichnet, dass**
n = 1-5, bevorzugt n = 1 oder n = 2,
R¹ = (C₁-C₄)-Alkyl, (C₆-C₁₀)-Aryl, (C₇-C₁₃)-Aralkyl, (C₇-C₁₀)-Heteroaralkyl, Pyridyl, Hydroxymethyl, CH(OH)CH₃, CH₂CONH₂, CH₂COOH, (CH₂)₂CONH₂, (CH₂)₂COOH, (CH₂)₄NH₂, (CH₂)₂SCH₃ oder (CH₂)₃NHC(NH)NH₂,
wobei entweder X = OH ist oder einen Aminopropyllinker darstellt, über den der Selektor als Carbonsäureamid an das Trägermaterial gebunden ist,
und R² = 3,5-Dinitrobenzoyl oder Naphthyl bedeutet, wenn X einen Linker zur kovalenten Anbindung an ein Trägermaterial darstellt,
oder R² = CH₂CHR³R⁴ bedeutet, worin R³ = H oder OH und R⁴ = (C₁-C₂₀)-Alkyl, (C₆-C₁₀)-Aryl oder (C₇-C₁₃)-Aralkyl bedeutet, wenn X = OH darstellt.

2. Verwendung von β-Aminosäurederivaten gemäß Anspruch 1, **dadurch gekennzeichnet, dass** der chirale Selektor in überwiegend einer absoluten Konfiguration bezüglich des Kohlenstoffatomes C-3 (β-Kohlenstoffatom) vorliegt.

3. Verwendung von β-Aminosäurederivaten gemäß Anspruch 1 oder 2, **dadurch gekennzeichnet, dass** R¹ = Phenyl bedeutet.

4. Verwendung von β-Aminosäurederivaten gemäß irgendeinem der Ansprüche 1 bis 3, **dadurch gekennzeichnet, dass** R⁴ = (C₈-C₁₈)-Alkyl, bevorzugt (C₆-C₁₆)-Alkyl, insbesondere (C₁₀-C₁₂)-Alkyl und besonders bevorzugt R⁴ = n-Decyl bedeutet.

5. Verwendung von β-Aminosäurederivaten gemäß irgendeinem der Ansprüche 1 bis 3, **dadurch gekennzeichnet, dass** R⁴ = Phenyl und n = 1 bedeutet.

6. Verwendung von β-Aminosäurederivaten der Struktur 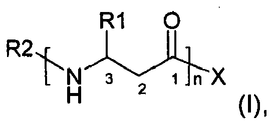 wobei
n = 1-5, bevorzugt n = 1 oder n = 2,
R¹ = (C₁-C₄)-Alkyl, (C₆-C₁₀)-Aryl, (C₇-C₁₃)-Aralkyl, (C₇-C₁₀)-Heteroaralkyl, Pyridyl, Hydroxymethyl, CH(OH)CH₃, CH₂CONH₂, CH₂COOH, (CH₂)₂CONH₂, (CH₂)₂COOH, (CH₂)₄NH₂, (CH₂)₂SCH₃ oder (CH₂)₃NHC(NH)NH₂,
wobei ferner entweder X = OH ist oder einen Aminopropyllinker darstellt, über den der Selektor als Carbonsäureamid an das Trägermaterial gebunden ist,
und R² = 3,5-Dinitrobenzoyl oder Naphthyl bedeutet, wenn X einen Linker zur kovalenten Anbindung an ein Trägermaterial darstellt,
oder R² = CH₂CHR³R⁴ bedeutet, worin R³ = H oder OH und R⁴ = (C₁-C₂₀)-Alkyl, (C₆-C₁₀)-Aryl oder (C₇-C₁₃)-Aralkyl bedeutet, wenn X = OH darstellt zur Herstellung stationärer Phasen für chromatographische Trennverfahren von Substanzgemischen, bevorzugt von Gemischen chiraler Substanzen, besonders bevorzugt von Enantiomeren, insbesondere von Enantiomeren von Substanzen ausgewählt aus der Gruppe enthaltend β-Aminosäuren und ihre Derivate, α-Aminosäuren und α-Hydroxysäuren, **dadurch gekennzeichnet, dass** der chirale Selektor kovalent oder adsorptiv an ein Trägermaterial auf Kieselgel- oder Monolithbasis gebunden wird.

7. Eine chirale stationäre Phase zur chromatographischen Trennung von Substanzgemischen, bevorzugt von Gemischen chiraler Substanzen, besonders bevorzugt von Enantiomeren, insbesondere von Enantiomeren von Substanzen ausgewählt aus der Gruppe enthaltend β-Aminosäuren und ihre Derivate, α-Aminosäuren und α-Hydroxysäuren, beinhaltend ein Trägermaterial und einen chiralen Selektor, **dadurch gekennzeichnet, dass** der chirale Selektor ein in α-Position unsubstituiertes □β-Aminosäurederivat der Struktur 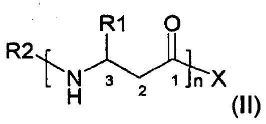 beinhaltet, worin n = 1-5, bevorzugt n = 1 oder n = 2, R¹ = (C₁-C₄)-Alkyl, (C₆-C₁₀)-Aryl, (C₇-C₁₃)-Aralkyl, (C₇-C₁₀)-Heteroaralkyl, Pyridyl, Hydroxymethyl, CH(OH)CH₃, CH₂CONH₂, CH₂COOH, (CH₂)₂CONH₂, (CH₂)₂COOH, (CH₂)₄-NH₂, (CH₂)₂-SCH₃ oder (CH₂)₃NHC(NH)NH₂, R² = 3,5-Dinitrobenzoyl oder Naphthyl bedeutet und X einen Aminopropyllinker darstellt, über den der Selektor als Carbonsäureamid an das Trägermaterial gebunden ist.

8. Eine chirale stationäre Phase gemäß Anspruch 7, **dadurch gekennzeichnet, dass** der chirale Selektor in überwiegend einer absoluten Konfiguration bezüglich des Kohlenstoffatomes C-3 (β-Kohlenstoffatom) vorliegt.

9. Eine chirale stationäre Phase gemäß Anspruch 7 oder 8, **dadurch gekennzeichnet, dass** R¹ = Phenyl darstellt.

10. Eine chirale stationäre Phase gemäß irgendeinem der Ansprüche 7 bis 9, **dadurch gekennzeichnet, dass** das Trägermaterial eine Kieselgel- oder Monolithphase ist.

11. Verfahren zur Herstellung einer chiralen stationären Phase gemäß irgendeinem der Ansprüche 7 bis 10 umfassend die folgenden Schritte:
(ia) Acylierung (R² = 3,5-Dinitrobenzoyl) einer in α-Position unsubstituierten β-Aminosäure oder des entsprechenden β-Aminosäureesters mit einem zur Acylierung geeigneten 3,5-Dinitrobenzoylderivat, bevorzugt 3,5-Dinitrobenzoylchlorid oder
(ib) Synthese einer in α-Position unsubstituierten N-naphthylierten β-Aminosäure oder des entsprechenden β-Aminosäureesters (R² = Naphthyl)
(ii) gegebenenfalls Verseifung der Esterfunktion,
(iii) gegebenenfalls Kupplung weiterer in α-Position unsubstituierter β-Aminosäureeinheiten,
(iv) kovalente Anbindung des chiralen Selektors an ein Trägermaterial, mittels eines Aminopropyllinkers, über den der Selektor als Carbonsäureamid an das Trägermaterial gebunden ist.

12. Verfahren zur chromatographischen Trennung von Substanzgemischen, bevorzugt von Gemischen chiraler Substanzen, besonders bevorzugt von Enantiomeren, insbesondere von Enantiomeren von Substanzen ausgewählt aus der Gruppe enthaltend β-Aminosäuren und ihre Derivate, α-Aminosäuren und α-Hydroxysäuren, umfassend die folgenden Schritte:
(i) In-Kontakt-Bringen einer Lösung des zu trennenden Substanzgemisches mit einer chiralen stationären Phase gemäß irgendeinem der Ansprüche 7 bis 10,
(ii) Auftrennung der einzelnen Bestandteile des Substanzgemisches aufgrund ihrer unterschiedlichen Wechselwirkung mit der stationären Phase unter Verwendung einer mobilen Phase,
(iii) Gegebenenfalls das fraktionierende Sammeln der mobilen Phase und die Isolierung der chromatographisch gereinigten Substanzen hieraus.

13. Verfahren gemäß Anspruch 12 zur Minorspurenbestimmung von Enantiomeren, bevorzugt von Enantiomeren von α- und β-Aminosäuren.

14. Verfahren gemäß Anspruch 12 oder 13, **dadurch gekennzeichnet, dass** es sich um flüssigchromatographische Verfahren, bevorzugt HPLC-Verfahren, handelt.

15. Eine chirale stationäre Phase zur chromatographischen Trennung von Substanzgemischen, bevorzugt von Gemischen chiraler Substanzen, besonders bevorzugt von Enantiomeren, insbesondere von Enantiomeren von Substanzen ausgewählt aus der Gruppe enthaltend β-Aminosäuren und ihre Derivate, α-Aminosäuren und α-Hydroxysäuren, beinhaltend ein Trägermaterial und einen chiralen Selektor, **dadurch gekennzeichnet, dass** der chirale Selektor ein in α-Position unsubstituiertes □β-Aminosäurederivat der Struktur 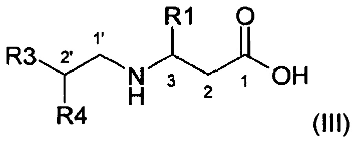 darstellt, worin R¹ = (C₁-C₄)-Alkyl, (C₆-C₁₀)-Aryl, (C₇-C₁₃)-Aralkyl, (C₇-C₁₀)-Heteroaralkyl, Pyridyl, Hydroxymethyl, CH(OH)CH₃, CH₂CONH₂. CH₂COOH, (CH₂)₂CONH₂, (CH₂)₂COOH, (CH₂)₄-NH₂, (CH₂)₂-SCH₃ oder (CH₂)₃NHCNHNH₂ und R³ = H oder OH und R⁴ = (C₁-C₂₀)-Alkyl, (C₆-C₁₀)-Aryl oder (C₇-C₁₃)-Aralkyl bedeutet.

16. Eine chirale stationäre Phase gemäß Anspruch 15, **dadurch gekennzeichnet, dass** der chirale Selektor in überwiegend einer absoluten Konfiguration bezüglich des Kohlenstoffatomes C-3 (β-Kohlenstoffatom) vorliegt, während das in 2'-Position potentiell vorhandene Stereozentrum nicht in überwiegend einer absoluten Konfiguration vorliegen muss.

17. Eine chirale stationäre Phase gemäß Anspruch 15 oder 16, **dadurch gekennzeichnet, dass** R¹ = Phenyl bedeutet.

18. Eine chirale stationäre Phase gemäß irgendeinem der Ansprüche 15 bis 17, **dadurch gekennzeichnet, dass** R⁴ = (C₄-C₁₈)-Alkyl, bevorzugt (C₆-C₁₆)-Alkyl, insbesondere (C₁₀-C₁₂)-Alkyl und besonders bevorzugt R⁴ = n-Decyl bedeutet.

19. Eine chirale stationäre Phase gemäß irgendeinem der Ansprüche 15 bis 17, **dadurch gekennzeichnet, dass** R⁴ = Phenyl bedeutet.

20. Eine chirale stationäre Phase gemäß irgendeinem der Ansprüche 15 bis 19, **dadurch gekennzeichnet, dass** der chirale Selektor adsorptiv an ein Trägermaterial auf Kieselgel- oder Monolithbasis gebunden ist.

21. Eine chirale stationäre Phase gemäß Anspruch 20, **dadurch gekennzeichnet, dass** das Trägermaterial ein Reversed-Phase-Material ist.

22. Eine chirale stationäre Phase gemäß Anspruch 21, **dadurch gekennzeichnet, dass** das Trägermaterial ausgewählt ist aus der Gruppe umfassend die Phasen RP-2, RP-4, RP-5, RP-6, RP-8, RP-12 und insbesondere RP-18.

23. Verfahren zur Herstellung einer chiralen stationären Phase gemäß irgendeinem der Ansprüche 15 bis 22 umfassend die folgenden Schritte:
(i) Darstellung der N-alkylierten Aminosäure,
(ii) adsorptive Anbindung der N-alkylierten Aminosäure an ein Trägermaterial.

24. Verfahren zur chromatographischen Trennung von Substanzgemischen, bevorzugt von Gemischen chiraler Substanzen, besonders bevorzugt von Enantiomeren, insbesondere von Enantiomeren von Substanzen ausgewählt aus der Gruppe enthaltend β-Aminosäuren und ihre Derivate, α-Aminosäuren und α-Hydroxysäuren, umfassend die folgenden Schritte:
(i) In-Kontakt-Bringen einer Lösung des zu trennenden Substanzgemisches mit einer chiralen stationären Phase beinhaltend einen chiralen Selektor gemäß irgendeinem der Ansprüche 15 bis 22,
(ii) Auftrennung der einzelnen Bestandteile des Substanzgemisches aufgrund ihrer unterschiedlichen Wechselwirkung mit dem chiralen Selektor unter Verwendung einer mobilen Phase enthaltend zweiwertige Übergangsmetallionen, bevorzugt Kupfer(II)ionen,
(iii) gegebenenfalls das fraktionierende Sammeln der mobilen Phase und die Isolierung der chromatographisch gereinigten Substanzen hieraus.

25. Verfahren gemäß Anspruch 24 zur Minorspurenbestimmung von Enantiomeren, bevorzugt von Enantiomeren von α- und β-Aminosäuren.

## Claims

1. Use of α-unsubstituted β-amino acid derivatives of the structure 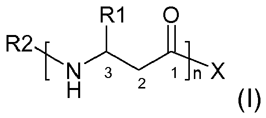 as chiral selectors in processes for separating substance mixtures, preferably mixtures of chiral substances, more preferably enantiomers, especially enantiomers of substances selected from the group comprising β-amino acids and derivatives thereof, α-amino acids and α-hydroxy acids, **characterized in that**
n = 1-5, preferably n = 1 or n = 2,
R¹ = (C₁-C₄)-alkyl, (C₆-C₁₀)-aryl, (C₇-C₁₃)-aralkyl, (C₇-C₁₀)-heteroaralkyl, pyridyl, hydroxymethyl, CH(OH)CH₃, CH₂CONH₂, CH₂COOH, (CH₂)₂CONH₂, (CH₂)₂COOH, (CH₂)₄NH₂, (CH₂)₂SCH₃ or (CH₂)₃NHC(NH)NH₂
where either X = OH or an aminopropyl linker through which the selector is bonded as a carboxamide to the support material,
and R² = 3,5-dinitrobenzoyl or naphthyl when X is a linker to the covalent attachment to a support material,
or R² = CH₂CHR³R⁴ in which R³ = H or OH and R⁴ = (C₁-C₂₀)-alkyl, (C₆-C₁₀)-aryl or (C₇-C₁₃)-aralkyl when X = OH.

2. Use of β-amino acid derivatives according to Claim 1, **characterized in that** the chiral selector is present in predominantly one absolute configuration with respect to the C-3 carbon atom (β-carbon atom).

3. Use of β-amino acid derivatives according to Claim 1 or 2, **characterized in that** R¹ = phenyl.

4. Use of β-amino acid derivatives according to any one of Claims 1 to 3, **characterized in that** R⁴ = (C₈-C₁₈)-alkyl, preferably (C₆-C₁₆)-alkyl, especially (C₁₀-C₁₂)-alkyl, and, more preferably, R⁴ = n-decyl.

5. Use of β-amino acid derivatives according to any one of Claims 1 to 3, **characterized in that** R⁴ = phenyl and n = 1.

6. Use of β-amino acid derivatives of the structure 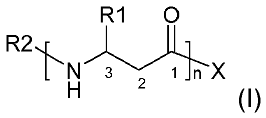 where
n = 1-5, preferably n = 1 or n = 2,
R¹ = (C₁-C₄)-alkyl, (C₆-C₁₀)-aryl, (C₇-C₁₃)-aralkyl, (C₇-C₁₀)-heteroaralkyl, pyridyl, hydroxymethyl, CH(OH)CH₃, CH₂CONH₂, CH₂COOH, (CH₂)₂CONH₂, (CH₂)₂COOH, (CH₂)₄NH₂, (CH₂)₂SCH₃ or (CH₂)₃NHC(NH)NH₂,
where, in addition, X = OH or an aminopropyl linker through which the selector is bonded as a carboxamide to the support material,
and R² = 3,5-dinitrobenzoyl or naphthyl when X is a linker to the covalent attachment to a support material,
or R² = CH₂CHR³R⁴ in which R³ = H or OH and R⁴ = (C₁-C₂₀)-alkyl, (C₆-C₁₀)-aryl or (C₇-C₁₃)-aralkyl when X = OH,
for preparing stationary phases for chromatographic separation processes of substance mixtures, preferably mixtures of chiral substances, more preferably enantiomers, especially enantiomers of substances selected from the group comprising β-amino acids and derivatives thereof, α-amino acids and α-hydroxy acids, **characterized in that** the chiral selector is bonded covalently or adsorptively to a support material based on silica gel or a monolith.

7. Chiral stationary phase for chromatographic separation of substance mixtures, preferably mixtures of chiral substances, more preferably enantiomers, especially enantiomers of substances selected from the group comprising β-amino acids and derivatives thereof, α-amino acids and α-hydroxy acids, comprising a support material and a chiral selector, **characterized in that** the chiral selector is an α-unsubstituted β-amino acid derivative of the structure 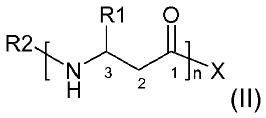 in which n = 1-5, preferably n = 1 or n = 2, R¹ = (C₁-C₄)-alkyl, (C₆-C₁₀)-aryl, (C₇-C₁₃)-aralkyl, (C₇-C₁₀)-heteroaralkyl, pyridyl, hydroxymethyl, CH(OH)CH₃, CH₂CONH₂, CH₂COOH, (CH₂)₂CONH₂, (CH₂)₂COOH, (CH₂)₄-NH₂, (CH₂)₂-SCH₃ or (CH₂)₃NHC(NH)NH₂, R² = 3,5-dinitrobenzoyl or naphthyl, and X is an aminopropyl linker through which the selector is bonded to the support material as a carboxamide.

8. Chiral stationary phase according to Claim 7, **characterized in that** the chiral selector is present in predominantly one absolute configuration with respect to the C-3 carbon atom (β-carbon atom).

9. Chiral stationary phase according to Claim 7 or 8, **characterized in that** R¹ = phenyl.

10. Chiral stationary phase according to any one of Claims 7 to 9, **characterized in that** the support material is a silica gel phase or a monolith phase.

11. Process for preparing a chiral stationary phase according to any one of Claims 7 to 10, comprising the following steps:
(ia) acylating (R² = 3,5-dinitrobenzoyl) an α-unsubstituted β-amino acid or the corresponding β-amino acid ester with a 3,5-dinitrobenzoyl derivative suitable for acylation, preferably 3,5-dinitrobenzoyl chloride, or
(ib) synthesizing an α-unsubstituted N-naphthylated β-amino acid or the corresponding β-amino acid ester (R² = naphthyl)
(ii) if appropriate hydrolyzing the ester function,
(iii) if appropriate coupling further α-unsubstituted β-amino acid units,
(iv) covalently attaching the chiral selector to a support material by means of an aminopropyl linker through which the selector is bonded to the support material as a carboxamide.

12. Process for chromatographically separating substance mixtures, preferably mixtures of chiral substances, more preferably enantiomers, especially enantiomers of substances selected from the group comprising β-amino acids and derivatives thereof, α-amino acids and α-hydroxy acids, comprising the following steps:
(i) contacting a solution of the substance mixture to be separated with a chiral stationary phase according to any one of Claims 7 to 10,
(ii) separating the individual constituents of the substance mixture on the basis of their different interaction with the stationary phase using a mobile phase,
(iii) if appropriate the fractional collection of the mobile phase and the isolation of the chromatographically purified substances therefrom.

13. Process according to Claim 12 for minor trace determination of enantiomers, preferably of enantiomers of α- and β-amino acids.

14. Process according to Claim 12 or 13, **characterized in that** it is a liquid chromatography process, preferably HPLC process.

15. Chiral stationary phase for chromatographic separation of substance mixtures, preferably of mixtures of chiral substances, more preferably of enantiomers, especially of enantiomers of substances selected from the group comprising β-amino acids and derivatives thereof, α-amino acids and α-hydroxy acids, comprising a support material and a chiral selector, **characterized in that** the chiral selector is an α-unsubstituted β-amino acid derivative of the structure 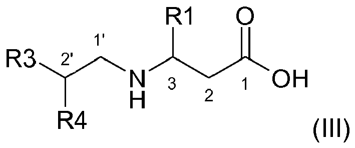 in which R¹ = (C₁-C₄)-alkyl, (C₆-C₁₀)-aryl, (C₇-C₁₃)-aralkyl, (C₇-C₁₀)-heteroaralkyl, pyridyl, hydroxymethyl, CH(OH)CH₃, CH₂CONH₂, CH₂COOH, (CH₂)₂CONH₂, (CH₂)₂COOH, (CH₂)₄-NH₂, (CH₂)₂-SCH₃ or (CH₂)₃NHCNHNH₂ and R³ = H or OH and R⁴ = (C₁-C₂₀)-alkyl, (C₆-C₁₀)-aryl or (C₇-C₁₃)-aralkyl.

16. Chiral stationary phase according to Claim 15, **characterized in that** the chiral selector is present in predominantly one absolute configuration with respect to the C-3 carbon atom (β-carbon atom), while the stereocenter potentially present in the 2' position need not be present in predominantly one absolute configuration.

17. Chiral stationary phase according to Claim 15 or 16, **characterized in that** R¹ = phenyl.

18. Chiral stationary phase according to any one of Claims 15 to 17, **characterized in that** R⁴ = (C₄-C₁₈)-alkyl, preferably (C₆-C₁₆)-alkyl, especially (C₁₀-C₁₂)-alkyl, and, more preferably, R⁴ = n-decyl.

19. Chiral stationary phase according to any one of Claims 15 to 17, **characterized in that** R⁴ = phenyl.

20. Chiral stationary phase according to any one of Claims 15 to 19, **characterized in that** the chiral selector is bonded adsorptively to a support material based on silica gel or a monolith.

21. Chiral stationary phase according to Claim 20, **characterized in that** the support material is a reversed-phase material.

22. Chiral stationary phase according to Claim 21, **characterized in that** the support material is selected from the group comprising the RP-2, RP-4, RP-5, RP-6, RP-8, RP-12 and especially RP-18 phases.

23. Process for preparing a chiral stationary phase according to any one of Claims 15 to 22, comprising the following steps:
(i) preparing the N-alkylated amino acid,
(ii) adsorptively attaching the N-alkylated amino acids to a support material.

24. Process for chromatographically separating substance mixtures, preferably mixtures of chiral substances, more preferably enantiomers, especially enantiomers of substances selected from the group comprising β-amino acids and derivatives thereof, α-amino acids and α-hydroxy acids, comprising the following steps:
(i) contacting a solution of the substance mixture to be separated with a chiral stationary phase comprising a chiral selector according to any one of Claims 15 to 22,
(ii) separating the individual constituents of the substance mixture on the basis of their different interaction with the chiral selector using a mobile phase comprising divalent transition metal ions, preferably copper(II) ions,
(iii) if appropriate the fractional collection of the mobile phase and the isolation of the chromatographically purified substances therefrom.

25. Process according to Claim 24 for minor trace determination of enantiomers, preferably of enantiomers of α- and β-amino acids.

## Revendications

1. Utilisation de dérivés de β-aminoacides non substitués en position α de structure 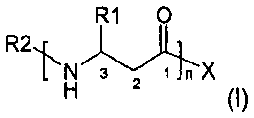 comme sélecteurs chiraux dans des procédés pour la séparation de mélanges de substances, de préférence de mélanges de substances chirales, de manière particulièrement préférée d'énantiomères, en particulier d'énantiomères de substances choisies dans le groupe contenant les β-aminoacides et leurs dérivés, les α-aminoacides et les α-hydroxyacides, **caractérisée en ce que**
n = 1-5, de préférence n = 1 ou n = 2,
R¹ = (C₁-C₄)-alkyle, (C₆-C₁₀)-aryle, (C₇-C₁₃)-aralkyle, (C₇-C₁₀)-hétéroaralkyle, pyridyle, hydroxyméthyle, CH(OH)CH₃, CH₂CONH₂, CH₂COOH, (CH₂)₂CONH₂, (CH₂)₂COOH, (CH₂)₄NH₂, (CH₂)₂SCH₃ ou (CH₂)₃NHC (NH) NH₂,
où soit X = OH, soit représente un lieur aminopropyle, via lequel le sélecteur est lié en tant qu'amide d'acide carboxylique au matériau support, et
R² = 3,5-dinitrobenzoyle ou naphtyle, lorsque X représente un lieur pour la liaison covalente à un matériau support, ou
R² = signifie CH₂CHR³R⁴, où R³ = H ou OH et R⁴ = (C₁-C₂₀)-alkyle, (C₆-C₁₀)-aryle ou (C₇-C₁₃)-aralkyle, lorsque X = OH.

2. Utilisation de dérivés de β-aminoacides selon la revendication 1, **caractérisée en ce que** le sélecteur chiral se trouve principalement dans une configuration absolue en ce qui concerne l'atome de carbone C-3 (atome de carbone en position β).

3. Utilisation de dérivés de β-aminoacides selon la revendication 1 ou 2, **caractérisée en ce que** R¹ = phényle.

4. Utilisation de dérivés de β-aminoacides selon l'une quelconque des revendications 1 à 3, **caractérisée en ce que** R⁴ = (C₈-C₁₈)-alkyle, de préférence (C₆-C₁₆)-alkyle, en particulier (C₁₀-C₁₂)-alkyle et de manière particulièrement préférée R⁴ = n-décyle.

5. Utilisation de dérivés de β-aminoacides selon l'une quelconque des revendications 1 à 3, **caractérisée en ce que** R⁴ = phényle et n = 1.

6. Utilisation de dérivés de β-aminoacides de structure 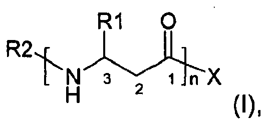 où
n = 1-5, de préférence n = 1 ou n = 2,
R¹ = (C₁-C₄)-alkyle, (C₆-C₁₀)-aryle, (C₇-C₁₃)-aralkyle, (C₇-C₁₀)-hétéroaralkyle, pyridyle, hydroxyméthyle, CH(OH)CH₃, CH₂CONH₂, CH₂COOH, (CH₂)₂CONH₂, (CH₂)₂COOH, (CH₂)₄NH₂, (CH₂)₂SCH₃ ou (CH₂)₃NHC (NH) NH₂,
où en outre soit X = OH, soit représente un lieur aminopropyle, via lequel le sélecteur est lié en tant qu'amide d'acide carboxylique au matériau support, et
R² = 3,5-dinitrobenzoyle ou naphtyle, lorsque X représente un lieur pour la liaison covalente sur un matériau support, ou
R² = signifie CH₂CHR³R⁴, où R³ = H ou OH et R⁴ = (C₁-C₂₀) - alkyle, (C₆-C₁₀)-aryle ou (C₇-C₁₃)-aralkyle, lorsque X = OH
pour la préparation de phases stationnaires pour des procédés de séparation chromatographiques de mélanges de substances, de préférence de mélanges de substances chirales, de manière particulièrement préférée d'énantiomères, en particulier d'énantiomères de substances choisies dans le groupe contenant les β-aminoacides et leurs dérivés, les α-aminoacides et les α-hydroxyacides, **caractérisée en ce que** le sélecteur chiral est lié par covalence ou par adsorption à un matériau support à base de gel silicique ou d'un monolithe.

7. Phase stationnaire chirale pour la séparation chromatographique de mélanges de substances, de préférence de mélanges de substances chirales, de manière particulièrement préférée d'énantiomères, en particulier d'énantiomères de substances choisies dans le groupe contenant les β-aminoacides et leurs dérivés, les α-aminoacides et les α-hydroxyacides, comprenant un matériau support et un sélecteur chiral, **caractérisée en ce que** le sélecteur chiral présente un dérivé de β-aminoacide non substitué en position α de structure 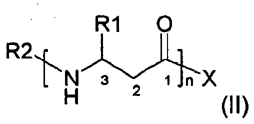 où n = 1-5, de préférence n = 1 ou n = 2, R¹ = (C₁-C₄)-alkyle, (C₆-C₁₀)-aryle, (C₇-C₁₃)-aralkyle, (C₇-C₁₀)-hétéroaralkyle, pyridyle, hydroxyméthyle, CH(OH)CH₃, CH₂CONH₂, CH₂COOH, (CH₂)₂CONH₂, (CH₂)₂COOH, (CH₂)₄-NH₂, (CH₂)₂-SCH₃ ou (CH₂)₃NHC(NH)NH₂, R² = 3,5-dinitrobenzoyle ou naphtyle et X représente un lieur aminopropyle, via lequel le sélecteur est lié en tant qu'amide d'acide carboxylique au matériau support.

8. Phase stationnaire chirale selon la revendication 7, **caractérisée en ce que** le sélecteur chiral se trouve principalement dans une configuration absolue en ce qui concerne l'atome de carbone C-3 (atome de carbone en position β).

9. Phase stationnaire chirale selon la revendication 7 ou 8, **caractérisée en ce que** R¹ = phényle.

10. Phase stationnaire chirale selon l'une quelconque des revendications 7 à 9, **caractérisée en ce que** le matériau support est une phase de gel silicique ou monolithique.

11. Procédé pour la préparation d'une phase stationnaire chirale selon l'une quelconque des revendications 7 à 10, comprenant les étapes suivantes :
(ia) acylation (R² = 3,5-dinitrobenzoyle) d'un β-aminoacide non substitué en position α ou de l'ester de β-aminoacide correspondant avec un dérivé de 3,5-dinitrobenzoyle approprié pour l'acylation, de préférence du chlorure de 3,5-dinitrobenzoyle ou
(ib) synthèse d'un β-aminoacide non substitué en position α, N-naphtylé ou de l'ester de β-aminoacide correspondant (R² = naphtyle)
(ii) le cas échéant saponification de la fonction ester,
(iii) le cas échéant couplage d'autres unités de β-aminoacide non substitué en position α,
(iv) liaison covalente du sélecteur chiral à un matériau support,
au moyen d'un lieur aminopropyle, via lequel le sélecteur est lié en tant qu'amide d'acide carboxylique au matériau support.

12. Procédé pour la séparation chromatographique de mélanges de substances, de préférence de mélanges de substances chirales, de manière particulièrement préférée d'énantiomères, en particulier d'énantiomères de substances choisies dans le groupe contenant les β-aminoacides et leurs dérivés, les α-aminoacides et les α-hydroxyacides, comprenant les étapes suivantes :
(i) mise en contact d'une solution du mélange de substances à séparer avec une phase stationnaire chirale selon l'une quelconque des revendications 7 à 10,
(ii) séparation des différents constituants du mélange de substances en raison de leur interaction différente avec la phase stationnaire en utilisant une phase mobile,
(iii) le cas échéant récupération fractionnée de la phase mobile et isolement des substances purifiées chromatiquement à partir de celle-ci.

13. Procédé selon la revendication 12 pour la détermination de traces mineures d'énantiomères, de préférence d'énantiomères d'α-aminoacides et de β-aminoacides.

14. Procédé selon la revendication 12 ou 13, **caractérisé en ce qu'**il s'agit de procédés chromatographiques en phase liquide, de préférence de procédés HPLC.

15. Phase stationnaire chirale pour la séparation chromatographique de mélanges de substances, de préférence de mélanges de substances chirales, de manière particulièrement préférée d'énantiomères, en particulier d'énantiomères de substances choisies dans le groupe contenant les β-aminoacides et leurs dérivés, les α-aminoacides et les α-hydroxyacides, comprenant un matériau support et un sélecteur chiral, **caractérisé en ce que** le sélecteur chiral présente un dérivé β-aminoacide non substitué en position α de structure 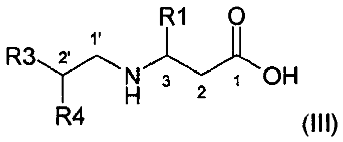 où R¹ = (C₁-C₄)-alkyle, (C₆-C₁₀)-aryle, (C₇-C₁₃)-aralkyle, (C₇-C₁₀)-hétéroaralkyle, pyridyle, hydroxyméthyle, CH(OH)CH₃, CH₂CONH₂, CH₂COOH, (CH₂)₂CONH₂, (CH₂)₂COOH, (CH₂)₄-NH₂, (CH₂)₂-SCH₃ ou (CH₂)₃NHCNHNH₂ et R³ = H ou OH et R⁴ = (C₁-C₂₀)-alkyle, (C₆-C₁₀)-aryle ou (C₇-C₁₃)-aralkyle.

16. Phase stationnaire chirale selon la revendication 15, **caractérisée en ce que** le sélecteur chiral se trouve principalement dans une configuration absolue en ce qui concerne l'atome de carbone C-3 (atome de carbone en position β) alors que le stéréocentre potentiellement présent en position 2' ne doit pas se trouver principalement dans une configuration absolue.

17. Phase stationnaire chirale selon la revendication 15 ou 16, **caractérisée en ce que** R¹ = phényle.

18. Phase stationnaire chirale selon l'une quelconque des revendications 15 à 17, **caractérisée en ce que** R⁴ = (C₄-C₁₈)-alkyle, de préférence (C₆-C₁₆)-alkyle, en particulier (C₁₀-C₁₂)-alkyle et de manière particulièrement préférée R⁴ = n-décyle.

19. Phase stationnaire chirale selon l'une quelconque des revendications 15 à 17, **caractérisée en ce que** R⁴ = phényle.

20. Phase stationnaire chirale selon l'une quelconque des revendications 15 à 19, **caractérisée en ce que** le sélecteur chiral est lié par adsorption sur un matériau support à base de gel silicique ou d'un monolithe.

21. Phase stationnaire chirale selon la revendication 20, **caractérisée en ce que** le matériau support est un matériau en phase inversée.

22. Phase stationnaire chirale selon la revendication 21, **caractérisée en ce que** le matériau support est choisi dans le groupe comprenant les phases RP-2, RP-4, RP-5, RP-6, RP-8, RP-12 et en particulier RP-18.

23. Procédé pour la préparation d'une phase stationnaire chirale selon l'une quelconque des revendications 15 à 22, comprenant les étapes suivantes :
(i) préparation de l'aminoacide N-alkylé,
(ii) liaison par adsorption de l'aminoacide N-alkylé sur un matériau support.

24. Procédé pour la séparation chromatographique de mélanges de substances, de préférence de mélanges de substances chirales, de manière particulièrement préférée d'énantiomères, en particulier d'énantiomères de substances choisies dans le groupe contenant les β-aminoacides et leurs dérivés, les α-aminoacides et les α-hydroxyacides, comprenant les étapes suivantes :
(i) mise en contact d'une solution du mélange de substances à séparer avec une phase stationnaire chirale comportant un sélecteur chiral selon l'une quelconque des revendications 15 à 22,
(ii) séparation des différents constituants du mélange de substances en raison de leur interaction différente avec le sélecteur chiral en utilisant une phase mobile contenant des ions divalents de métal de transition, de préférence des ions de cuivre (II),
(iii)le cas échéant récupération fractionnée de la phase mobile et isolement des substances purifiées chromatographiquement à partir de celle-ci.

25. Procédé selon la revendication 24 pour la détermination de traces mineures d'énantiomères, de préférence d'énantiomères d'α-aminoacides et de β-aminoacides.
